# EUROPÄISCHE PATENTSCHRIFT

(11) **EP 2 099 801 B1**
(45) Veröffentlichungstag und Bekanntmachung des Hinweises auf die Patenterteilung: **03.11.2010**
(21) Anmeldenummer: 07819823.1
(22) Anmeldetag: 15.11.2007
(51) Int. Cl.: C07D 487/04, B01J 31/00, C07B 35/02, C07B 41/02, C07B 53/00

(54) **IMIDAZOÝ1,5-B¨PYRIDAZIN-AMIDO-LIGANDEN UND DEREN KOMPLEXVERBINDUNGEN**
IMIDAZOÝ1,5-B¨PYRIDAZINAMIDO LIGANDS AND THEIR COMPLEXES
LIGANDS IMIDAZOÝ1,5-B¨PYRIDAZINE-AMIDO ET LEURS COMPOSÉS COMPLEXES

(30) Priorität: 23.11.2006 DE 102006055716
(43) Veröffentlichungstag der Anmeldung: 16.09.2009
(73) Patentinhaber: AIKAA-Chemicals-GmbH, 95444 Bayreuth (DE)
(72) Erfinder: KEMPE, Rhett, 95445 Bayreuth (DE); IRRGANG, Torsten, 95445 Bayreuth (DE); FRIEDRICH, Denise, 96274 Itzgund (DE)
(74) Vertreter: Ackermann, Joachim
(86) Internationale Anmeldenummer: PCT/EP2007/009875
(87) Internationale Veröffentlichungsnummer: WO 2008/061663

(56) Entgegenhaltungen:
- FR-A- 2 801 886
- IRRGANG T ET AL: "Early and Late Transition Metal Complexes Stabilised by Imidazopyridazine-Substituted Bisamido Ligands" EUROPEAN JOURNAL OF INORGANIC CHEMISTRY, WILEY-VCH VERLAG, WEINHEIM, DE, 2005, Seiten 4382-4392, XP003000910 ISSN: 1434-1948 in der Anmeldung erwähnt

## Beschreibung

Die Erfindung betrifft neue NN(R)-Liganden (Imidazo[1,5-*b*]pyridazin-substituierte Amino-Liganden) und damit herstellbare Katalysatoren, insbesondere zur asymmetrischen Katalyse sowie ein Verfahren zur Herstellung solcher Liganden und Katalysatoren.

Selektive Hydrierkatalysatoren beispielsweise für die (enantioselektive) Reduktion von Ketonen spielen in der Technik eine immer bedeutendere Rolle, insbesondere bei der enantioselektiven Hydrierung von Ketonen zu Alkoholen. Enantiomerenreine Alkohole, Amine und Aminosäuren haben als chirale Synthesebausteine eine herausragende technische Bedeutung z.B. bei der Erzeugung pharmazeutischer Wirkstoffe und Agrochemikalien. Beispiele für industriell bedeutende Zielstrukturen mit pharmakologischer Wirkung, die von derartigen chiralen Verbindungen abgeleitet sind, sind z.B. Antibiotika (Cytoxazone), Antidepressiva (1555U88), Hemmer der Farnesyltransferase (Kurasoin A), verschiedene antifungale Wirkstoffe (Ro 09-3355, Sch42426, SCH 42427) und antivirale Wirkstoffe (Saquinavir). Zur Herstellung der gewünschten enantiomerenreinen Verbindungen werden bis heute vor allem Chiral-Pool-Verfahren oder klassische Racematspaltungen verwendet. Während eine limitierte Anzahl von Chiral-Pool-Bausteinen zur Verfügung steht, ergeben Racematspaltungsverfahren prinzipiell nur 50% Ausbeute der gewünschten Produkte. Es besteht daher ein Bedarf an Ligandensystemen, die sich für asymmetrische Synthesen, z.B. zur enantioselektiven Hydrierung von Ketonen eignen.

Zur direkten enantioselektiven Herstellung werden bisher vor allem phosphorhaltige Katalysatorsysteme eingesetzt, deren Herstellung meist technisch aufwendig ist und die in der Regel oxidationsempfindlich sind. Solche klassischen, in der asymmetrischen Katalyse erfolgreich genutzten Liganden werden häufig durch komplizierte, mehrstufige Syntheseschritte erzeugt. Sehr häufig ist es hierzu notwendig, unter rigorosem Ausschluss von Luft (Feuchtigkeit und Sauerstoff) zu arbeiten. Diese Synthesevarianten sind technisch aufwendig, zeitintensiv und führen zu hohen Herstellungskosten für die resultierenden Liganden bzw. Katalysatorsysteme.

Leichter herzustellende deprotonierte Amine (Amidoverbindungen) sind hingegen als Liganden in der enantioselektiven homogenen Katalyse, insbesondere bei der enantioselektiven Hydrierung, bisher nicht in Erscheinung getreten. In T. Irrgang, R. Kempe, Eur. J. Inorg. Chem. 2005, 4382-4392 und T. Irrgang, Dissertation 2000, Greifswald werden weiterhin bestimmte neue Imidazopyridazin-bisamido-Liganden und optisch nicht aktive Imidazopyridazin-amino-hydroxy-Verbindungen beschrieben, die bisher allerdings nicht in der Katalyse zum Einsatz gekommen sind und im besonderen für eine enantioselektive Reaktionsführung aufgrund der mangelnden optischen Aktivität nicht geeignet sind.

Aufgabe der vorliegenden Erfindung ist es nun neue, einfach herzustellende Liganden sowie die entsprechenden katalytisch aktiven Komplexverbindungen bereitzustellen, die sich für eine möglichst gute enantioselektive Reaktionsführung, insbesondere zur asymmetrischen Hydrierung, mit guten Ausbeuten eignen.

Die Aufgabe wird durch NN(R)-Liganden der Formel **1** (Imidazo[1,5-*b*]pyridazin substituierte Amino-Liganden) gelöst, 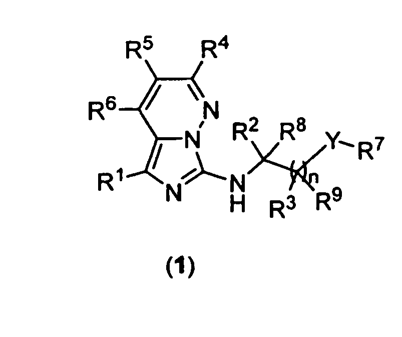 wobei
- n: für eine ganze Zahl von 1 bis 30, bevorzugt für eine Zahl von 1 bis 10, besonders bevorzugt von 1 bis 5, steht,
- Y: für ein Sauerstoff oder Schwefelatom oder eine NH-Gruppe steht,
- R¹-R⁹: unabhängig voneinander einen Rest ausgewählt aus der Gruppe C₁- C₂₄ Alkyl, C₂-C₂₄ Alkenyl, C₃-C₈ Cycloalkyl, C₃-C₈ Cycloalkenyl, C₆- C₁₄ Aryl, Phenyl, Naphthyl, Fluorenyl, C₃-C₈ Heteroalkyl/-cycloalkyl, C₂-C₁₃ Heteroaryl, Hydroxy, Acyl, Silyl, Boryl, darstellen können und wobei die Reste R¹, R⁴, R⁵, R⁶ und R⁷ unabhängig voneinander auch einen Wasserstoffrest darstellen können und wobei R² und R⁸ Wasserstoffreste sein können, wenn mindestens einer der Reste R³ und R⁹ kein Wasserstoffsubstituent ist bzw. R³ und R⁹ verschiedene Nichtwasserstoff-Reste darstellen und wobei R³ und R⁹ Wasserstoffreste sein können, wenn mindestens einer der Reste R² und R⁸ kein Wasserstoffsubstituent ist bzw. R² und R⁸ verschiedene Nichtwasserstoff-Reste darstellen und bei denen die genannten Substituenten R¹ bis R⁹ jeweils ein oder mehrere weitere Substituenten besitzen können, und wobei zwei benachbarte Substituenten R¹, R⁴, R⁵ oder R⁶ oder die Substituenten R², R³, R⁷, R⁸ und R⁹ auch verbrückt sein können und wobei die Liganden der Formel 1 bevorzugt neutral bzw. ionisch, insbesondere anionisch, also z.B. einfach oder mehrfach deprotoniert, vorliegen können.

Durch selektive Deprotonierung der sekundären Aminogruppe von Verbindungen der Formel **1** entstehen die entsprechenden Amide.

Y steht bevorzugt für ein Sauerstoffatom.

Bevorzugte (C₁-C₂₄) Alkyl-Substituenten R¹ bis R⁹ im Sinne der vorliegenden Erfindung sind verzweigte oder unverzweigte Alkylreste mit 1 bis 24 Kohlenstoffatomen, insbesondere mit 1 bis 10 Kohlenstoffatomen, besonders bevorzugt mit 1, 2 oder 3 bis 5 Kohlenstoffatomen, wie z. B. Methyl, Ethyl, n-Propyl, iso-Propyl, 1-Methylethyl, n-Butyl, iso-Butyl, 1-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, n-Heptyl, n-Octyl, n-Nonyl, n-Decyl.

Bevorzugte (C₂-C₂₄) Alkenyl-Substituenten R¹ bis R⁹ im Sinne der vorliegenden Erfindung sind Alkenylreste mit 2 bis 24 Kohlenstoffatomen, insbesondere mit 2 bis 10 Kohlenstoffatomen, besonders bevorzugt mit 2 oder 3 bis 5 Kohlenstoffatomen, wie z. B. Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 1-Heptenyl, 2-Heptenyl, 1-Octenyl oder 2-Octenyl.

Bevorzugte (C₃-C₈) Cycloalkylsubstituenten R¹ bis R⁹ sind substituierte und unsubstituierte Cyclopentyl-, Cyclohexyl-, Cycloheptylreste, Norbornyl und Adamantyl. Bevorzugte entsprechende Heterocycloalkylreste sind Cycloalkylreste, bei denen 1 bis 3 Kohlenstoffatome durch Heteroatome ausgewählt aus der Gruppe Stickstoff, Schwefel und Sauerstoff, ersetzt sind. Insbesondere enthalten solche Reste nur einen oder zwei gleiche oder zwei verschiedene Heteroatome. Bevorzugte Heterocyclen sind Tetrahydrofuranyl, Tetrahydrothiophenyl, Pyrrolidyl, 1,3-Dioxolanyl, 1,3-Dithiolanyl, Imidazolidyl, Tetrahydropyranyl, Tetrahydrothiopyranyl, Piperidyl, 1,4-Dioxanyl, 1,4-Dithianyl, Piperazyl, Oxepanyl, Thiepanyl und Azepanyl.

Bevorzugte Arylsubstituenten R¹ bis R⁹ sind solche mit 6 bis 12 Kohlenstoffatomen, besonders bevorzugt sind Phenyl, 2-Alkylphenyl, 3-Alkylphenyl, 4-Alkylphenyl, 2,6-Dialkylphenyl, 3,5-Dialkylphenyl, 3,4,5-Trialkylphenyl, 2-Alkoxyphenyl, 3-Alkoxyphenyl, 4-Alkoxyphenyl, 2,6-Dialkoxyphenyl, 3,5-Dialkoxyphenyl, 3,4,5-Trialkoxylphenyl oder 3,5-Dialkyl-4-alkoxyphenyl.

Bevorzugte C₂-C₁₃ Heteroarylsubstituenten R¹ bis R⁹ enthalten 1 bis 3 Heteroatome, bevorzugt aus der Gruppe Stickstoff, Sauerstoff und Schwefel. Besonders bevorzugt sind Furanyl, Thiophenyl, Pyrryl, Pyridyl und Pyrazyl.

Besonders bevorzugte Silylsubstituenten R¹ bis R⁹ sind Trimethylsilyl, tert.-Butyldiphenylsilyl, tert.-Butyldimethylsilyl und Triisopropylsilyl.

Besonders bevorzugt stellt R¹ einen Rest ausgewählt aus der Gruppe bestehend aus Phenyl, Alkyl, tert.-Butyl oder Heteroaryl dar, die gegebenenfalls weitere Substituenten tragen können.

Besonders bevorzugte Reste R⁴ und R⁶ sind unabhängig voneinander ausgewählt aus der Gruppe bestehend aus Phenyl, Alkyl (bevorzugt Methyl) und Trifluormethyl.

R⁵ ist in der bevorzugten Ausführungsform ein Wasserstoffsubstituent.

Bei R² und R⁸ handelt es sich bevorzugt um Reste ausgewählt aus der Gruppe der aromatischen Reste, wie z.B. Phenyl, der aliphatisch verzweigten oder unverzweigten Alkylreste, um Alkylarylreste (z.B. Benzyl) oder um Wasserstoffsubstituenten.

R³ und R⁹ sind bevorzugt Wasserstoffsubstituenten, Hydroxyreste, Ketoreste, Hydroxyalkylreste, aromatischen Reste, wie z.B. Phenyl, aliphatisch verzweigte oder unverzweigte Alkylreste oder Alkylarylreste, wie z.B. Alkylphenylreste.

R⁷ ist bevorzugt ein Wasserstoffsubstituent oder ein Acetylrest.

Weiterhin können die genannten Substituenten R¹ bis R⁹ von Verbindungen der Formel **1** jeweils ein oder mehrere weitere Substituenten besitzen, wobei bevorzugte Substituenten unabhängig voneinander aus der Gruppe C₁-C₁₂ Alkyl, C₂-C₁₂ Alkenyl, C₁-C₁₀ Halogenalkyl, C₃-C₈ Cycloalkyl, C₃-C₈ Cycloalkenyl, C₂-C₉ Heteroalkyl, C₁-C₉ Heteroalkenyl, C₆-C₁₄ Aryl, Phenyl, Naphthyl, Fluorenyl, C₂-C₇ Heteroaryl, C₁-C₁₀ Alkoxy, C₁-C₉ Trihalomethylalkyl, Trifluormethyl, Trichlormethyl, Fluoro, Chloro, Hydroxy, C₁-C₈ substituierte Amino der Formen mono-, di-, tri- C₁-C₈-Alkylamino oder C₂-C₈ Alkenylamino oder mono-, di-, tri- C₆-C₈ Arylamino oder Carboxyl, Carboxylato der Form COOR', wobei R' ein einwertiges Kation oder ein C₁-C₈ Alkyl darstellt, oder C₁-C₈-Acyloxy, ausgewählt werden können. Die Zahl der Heteroatome in Heteroverbindungen ausgewählt aus der Gruppe Stickstoff, Sauerstoff und Schwefel, beträgt bevorzugt 1 bis 3. Unter einer Halogenverbindung wird bevorzugt eine entsprechende Fluoro-, Chloro- oder Bromoverbindung verstanden.

Insbesondere sind optisch aktive Liganden der Formel 1 bevorzugt, bei denen ein Enantiomer angereichert ist. Besonders bevorzugt sind Ligandsysteme, bei denen die Enantiomerenanreicherung 90 %, insbesondere 95 %, besonders bevorzugt 99 % übersteigt.

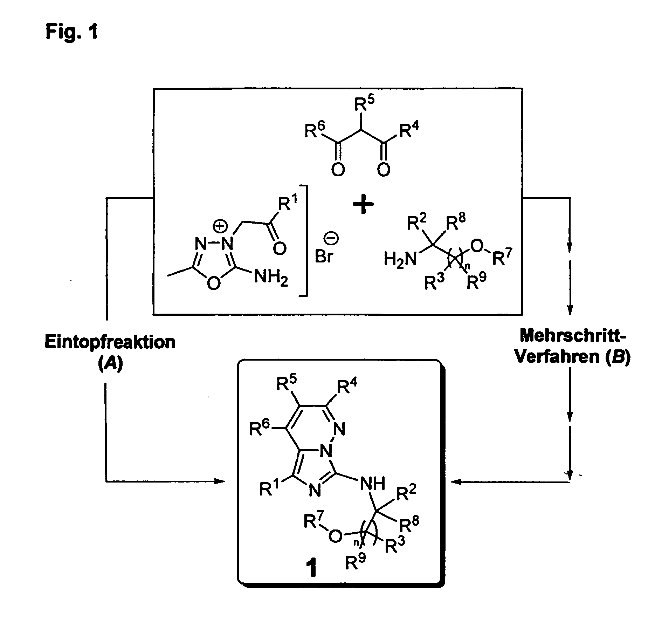

Die erfindungsgemäßen Liganden können ausgehend von preiswerten und in der Literatur beschriebenen (z.B. a) H. Beyer, A. Hetzheim, Chem. Ber. 1964, 97, 1031-1036; b) K. Peters, E.-M. Peters, A. Hetzheim, T. Irrgang, Z. Kristallogr. NCS 2000, 215, 381-381; c) T. Irrgang, *Dissertation* 2000, Greifswald) oder kommerziell erhältlichen Ausgangsmaterialien, bevorzugt in einem Einstufenprozess hergestellt werden. Dabei kann in Gegenwart von Luft gearbeitet werden, so dass ein ökonomisch und ökologisch sehr effizienter Zugang zu den protonierten NN(R)-Liganden der Formel **1**, ausgehenden von den beispielhaft gezeigten Edukten, möglich ist (siehe dazu auch Fig. 1, "Kipp-Schütt"-Chemie).

Im allgemeinen können die erfindungsgemäßen Liganden durch Umsetzung einer 2-Amino-3-acyl-oxadiazolium-verbindung I und einer Aminohydroxyverbindung II, z.B. durch einfaches Verreiben und Erhitzen, Aufnahme des entstandenen Zwischenproduktes in einem organischen Lösungsmittel, wie z.B. Ethanol, und Zugabe eines 1,3-Diketons **III** unter Ansäuern der Lösung, z.B. mit HCl, erhalten werden.

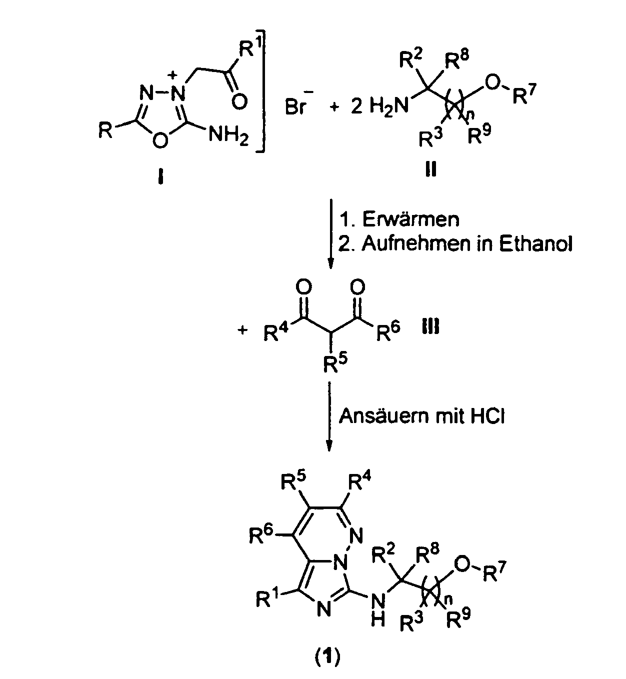

n, R¹ bis R⁹ haben dabei die oben genannte Bedeutung, der Rest R kann z.B. ein Wasserstoffsubstituent oder ein Alkylrest, bevorzugt ein Methylrest sein.

Im Falle das R² und R⁸ Wasserstoffsubstituenten und R³ oder R⁹ keine Wasserstoffsubstituenten sind liegt das Chiralitätszentrum an dem Kohlenstoffatom, an dem die Reste R³ und R⁹ gebunden sind.

Die erhaltenen Hydoxyliganden können im Anschluss einfach, mit dem Fachmann bekannten Verfahren, in die entsprechenden Thiol- oder Aminoverbindungen überführt werden.

So können z.B. die erfindungsgemäßen NN(R)-Liganden **1**, in protonierter bzw. deprotonierter Form, eine Vielzahl von Metallen und Elementen des Periodensystems der Elemente (PSE), insbesondere die der Gruppen 8, 9, 10 und 11, koordinieren und mit ihnen Komplexverbindungen bzw. Katalysatorsysteme ergeben. Ein weiterer Gegenstand der vorliegenden Erfindung sind daher bevorzugte Komplexverbindungen, der allgemeinen Formeln **2-6**, 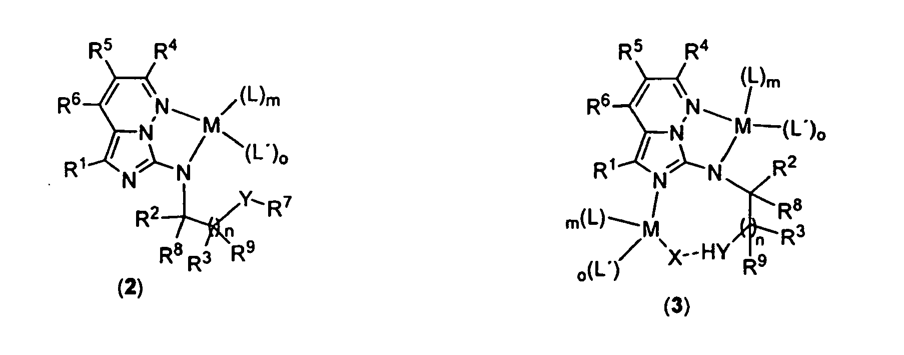 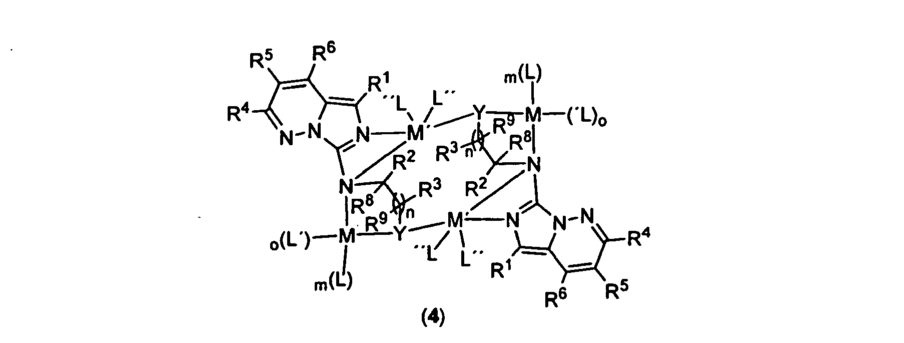 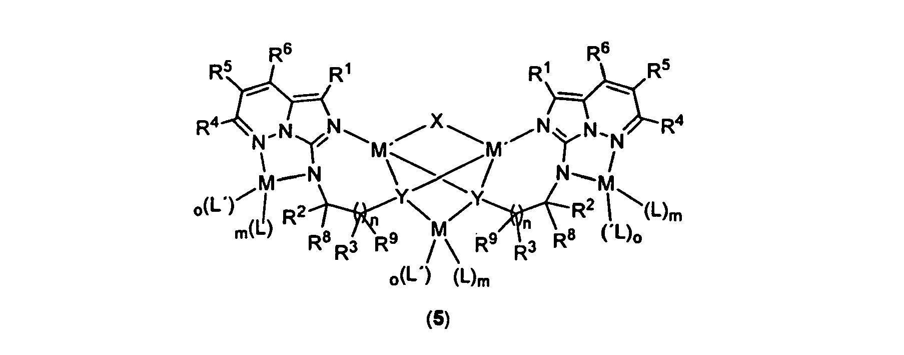 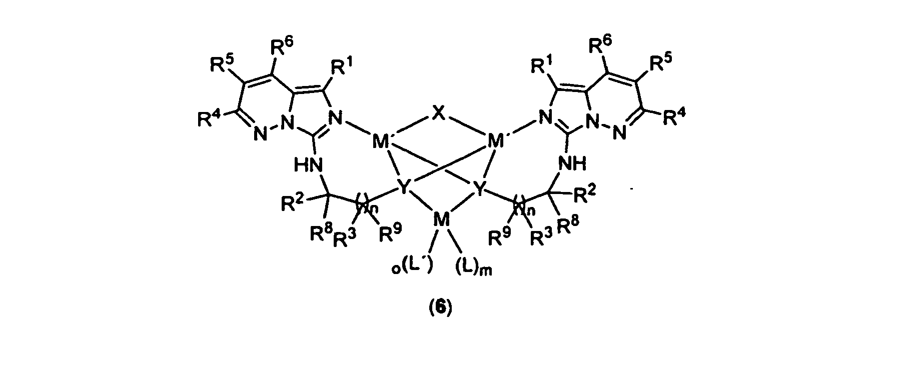 wobei
- n, Y und die: Substituenten R¹ bis R⁹ die oben genannte Bedeutung haben und
- m und o: unabhängig voneinander 0 oder 1 sein können, wobei bevorzugt m und o 1 sind, und
- X: für einen Halogenid-Liganden, insbesondere für einen Cl-, Br- und I- Liganden, steht und
- M: für ein Metall, bevorzugt ein Übergangsmetall aus der GruppeTi, Cr, Fe, Co, Ni, Cu, Zr, Ru, Rh, Pd, Os, Ir und Pt steht, besonders bevorzugte Metalle sind Iridium und Rhodium, und
- M': für ein Metall, bevorzugt ein Alkali- bzw. Erdalkalimetall steht, besonders bevorzugte Metalle sind Lithium, Natrium, Kalium und Magnesium, oder ein frühes Übergangsmetall ausgewählt aus der Gruppe Scandium, Yttrium, Lanthan, Titan, Zirkonium, Hafnium, oder ein Lanthanoid und
- L, L' und L": unabhängig voneinander für einen einzähnigen Liganden oder L und L' zusammen für einen zweizähnigen Liganden stehen.

Sofern in den Komplexen mehr als ein Metall M bzw. ein Metall M' enthalten ist, können die enthaltenen Metalle M genauso wie die Metalle M' unabhängig voneinander gleich oder verschieden sein.

Beispiele für L und L' sind u.a. Halogenid-Liganden, besonders Cl-, Br- und I-Liganden; Dien-Liganden, besonders Cyclopentadien, Cyclooctadien, Norbornadien; Olefin-Liganden, besonders Ethylen- und Cycloocten-Liganden, Acetato-; Trifluoracetato-; Acetylacetonato-; Allyl-; Methallyl-; Alkyl-Liganden; besonders Methyl- und Ethyl-Liganden; Nitril-Liganden, besonders Acetonitril- und Benzonitril-Liganden; Carbonyl- und Hydrido-Liganden; Phosphan-Liganden, beispielhaft Benzyldiphenylphosphan, t-Butyldicyclohexylphosphan, Tris(dimethylamido)phosphan, Cyclohexyldiphenylphosphan, Methyldiethoxyphosphan; sowie Phosphit-Liganden, beispielhaft Triisopropylphosphit, Trimethylphosphit, Tri-neo-pentylphosphit. L und L' stellen zusammengenommen besonders bevorzugt einen zweizähnigen Chelatliganden dar, insbesondere einen Cyclooctadien- oder Norbornadien-Liganden sowie Diphosphan-Liganden. Ausgewählte Beispiele für Diphosphan-Liganden sind 2,2'-Bis(di-*t*-butylphosphino)-1,1'-biphenyl, 1,3-Bis(di-*t*-butylphosphinomethyl)benzol, α,α'-Bis(di-*t*-butylphosphino)-o-xylol, 2,2'-Bis(dicyclohexylphosphino)-1,1'-biphenyl, 1,3-Bis(dicyclopentylphosphinomethyl)benzol, 1,2-Bis(diethylphosphino)ethan. Beispiele für L" sind u.a. Heterocycloalkyl-Liganden, besonders Tetrahydrofuran, Pyridin, 4-Methylpyridin, 4-*tert*.-Butylpyridin, Pyrimidin, Pyrazin, sowie Heteroalkyl-Liganden, besonders Diethylether.

Solche Komplexe **2-6** lassen sich z.B. in Gegenwart einer starken Base, bevorzugt in Gegenwart von Butyllithium, aus dem entsprechenden erfindungsgemäßen Liganden **1** und einem Metall, Metallvorkomplex oder Metallsalz in einem organischen Lösemittel herstellen.

Beispiele für die Metallsalze sind z.B. Metallchloride, -bromide, -iodide, -cyanide, - nitrate, -acetate, -acetylacetonate, -hexafluoracetylacetonate, tetrafluoroborate, - perfluoracetate oder -triflate, insbesondere des Palladiums, Platins, Rhodiums, Rutheniums, Osmiums, lridiums, Kobalts, des Nickels, des Eisens, des Kupfers, des Silbers und/oder des Golds.

Beispiele für die Metallvorkomplexe sind: 1,5-Hexadieniridiumchlorid oder -iodid, Cyclooctadieniridium(I)chlorid-Dimer, Bis(cycloocten)iridium(I)chlorid-Dimer, Cyclooctadieniridiumchlorid oder -iodid, Bis(ethylen)iridium(I)chlorid-Dimer, (Cyclooctadien)methoxyiridium(I) -Dimer, (Cyclooctadien)hydroxyiridium(I) -Dimer, Cyclooctadienrhodium(I)chlorid-Dimer, Norbornadienrhodium(I)chlorid-Dimer, 1,5-Hexadienrhodium(I)chlorid-Dimer, Tris(triphenylphosphan)rhodium(I)chlorid, Bis(ethylen)rhodium(I)chlorid-Dimer, (Cyclooctadien)methoxyrhodium(I) -Dimer, (Cyclooctadien)hydroxyrhodium(I) - Dimer, Hydridocarbonyltris(triphenylphosphan)rhodium(I)chlorid, Bis(cyclooctadien)rhodium(I)perchlorat, Bis(cyclooctadien)rhodium(I)tetrafluorborat, Bis(cyclooctadien)rhodium(I)triflat, Bis(acetonitril)(cyclooctadien)rhodium(I)perchlorat, -tetrafluorborat, oder -triflat, Cyclopentadienrhodium(III)chlorid-Dimer, Cyclooctadienpalladiumiodid oder - chlorid, 1,5-Hexadienpalladiumchlorid oder -iodid, Bis(dibenzylidenaceton)palladium, Bis(acetonitril)palladium(II)chlorid oder -bromid, Bis(benzonitril)palladium(II)chlorid, -bromid oder -iodid, Cyclooctadienplatinchlorid oder -iodid, 1,5-Hexadienplatinchlorid oder -iodid, Bis(cyclooctadien)platin, (Cyclooctadien)Ru(η³-allyl)₂, ((Cyclooctadien)Ru)₂(acetat)₄, ((Cyclooctadien)Ru)₂(trifluoracetat)₄, RuCl₂(Aren)-Dimer, Tris(triphenylphosphan)ruthenium(II)chlorid, Cyclooctadienruthenium(II)chlorid, OsCl₂(Aren)-Dimer, Bis(cyclooctadien)nickel, (Cyclododecatrien)nickel, Tris(norbornen)nickel, Nickeltetracarbonyl, Nickel(II)acetylacetonat, (Aren)kupfertriflat, (Aren)kupferperchlorat, (Aren)kupfertrifluoracetat, Kobaltcarbonyl.

Im Folgenden ist beispielhaft die Herstellung der erfindungsgemäßen NN(R)-Ligand-stabilisierten Komplexkatalysatoren **2'-6'** aus einem Iridium- bzw. Rhodiumvorkomplex via Salzeliminierung (**A**) bzw. Alkoholeliminierung (**B**) gezeigt:

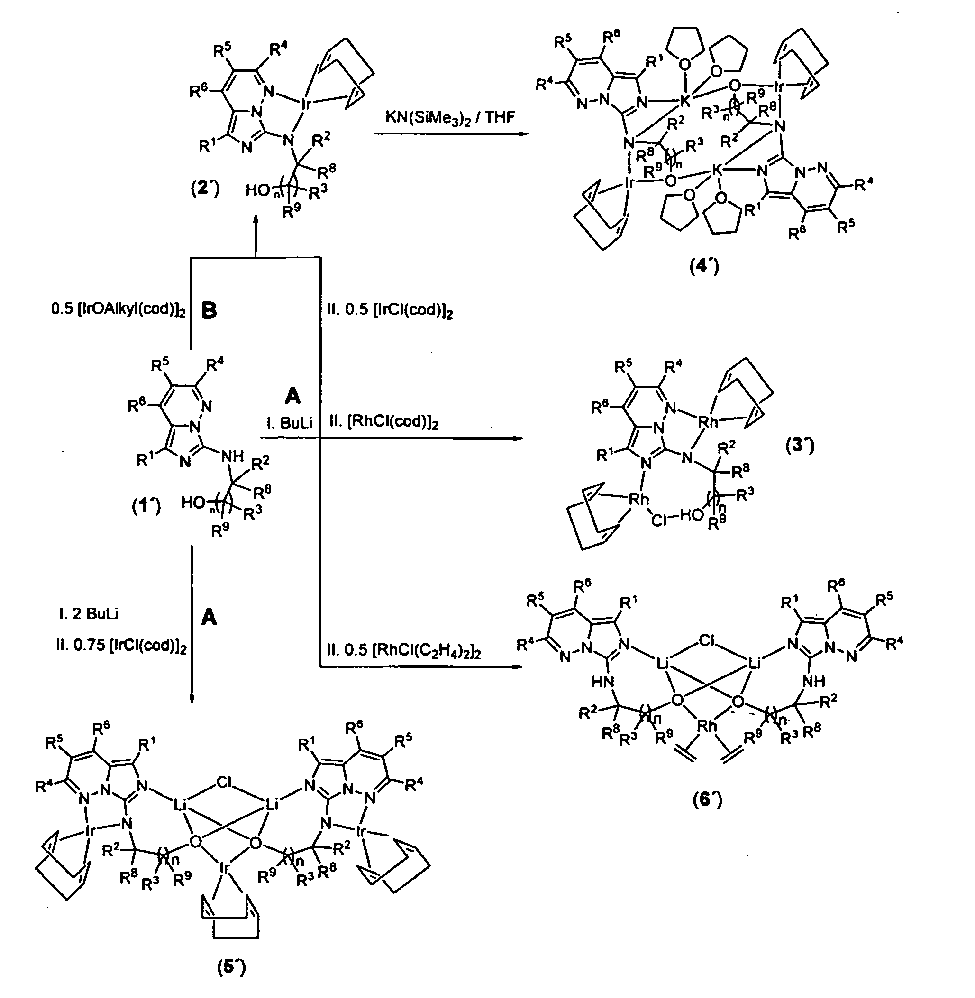

Der Syntheseweg **B** erlaubt die Generierung des Katalysators zur Hydrierung in einem Schritt (Isolierung und Aufreinigung des Katalysators entfällt). Dadurch wird das Verfahren zur Katalyse weiter vereinfacht und führt ebenfalls zu sehr guten Umsätzen und Enantioselektivitäten (siehe Beispiel 7a, Tabelle 1, Nr. 13-14 und Tabelle 3, Nr. 01-02; Beispiel 7d, Tabelle 9, Nr. 7 und Tabelle 10, Nr. 01-02). Katalysatoren, die die erfindungsgemäßen Liganden enthalten, werden bevorzugt zusammen mit ausgewählten Additiven eingesetzt. Bevorzugte Additive sind Metallalkoxidverbindungen, wie z.B. Alkali- oder Erdalkalialkoxide, bevorzugt KO^{t}Bu, NaO^{t}Bu oder M₉(OC₂H₅)₂, Alkali- oder Erdalkalimetall- alkyl-, allyl- und aryl-Verbindungen, wie z.B. BuLi, MeLi oder ^{t}BuLi, Alkali- oder Erdalkali-Phosphate, wie z.B. K₃PO₄, Alkali- oder Erdalkalihydride oder -halogenide, insbesondere deren Fluoride, wie z.B. KH, NaH, KF oder NaF, Alkali- oder Erdalkali-Silyloxyverbindungen oder entsprechende Amidoverbindungen, wie z.B. KOSiMe₃ oder KN(SiMe₃)₂, oder die Alkalimetalle selbst. Besonders bevorzugte Metallverbindungen sind in diesem Zusammenhang die entsprechenden Lithium-, Kalium- und Natriumverbindungen. Bevorzugt sind insbesondere deren Aryl-, Alkyl- und Allylverbindungen, deren Amidoverbindungen und deren Alkoholatverbindungen.

Komplexverbindungen der allgemeinen Formeln **2-6** sind insbesondere in Kombination mit Metallalkoxidverbindungen hochaktive und selektive Katalysatoren, insbesondere für Hydrierungen, wie beispielsweise für die (enantioselektive) Reduktion von Ketonen. Dabei entstehen chirale Alkohole, die auch einfach in die entsprechenden Amine überführt werden können. Durch die ungewöhnlich hohe Aktivität der Katalysatoren, die die beschriebenen Liganden enthalten, kann die Menge an eingesetztem Katalysator entsprechend verringert werden. Weiterhin sind mit den genannten Katalysatoren hohe Ausbeuten bei guter bis sehr guter Enantioselektivität zu beobachten.

Die erfindungsgemäßen Ligandensysteme besitzen eine Reihe technischer Vorteile, so sind sie einfach und preiswert zu synthetisieren. Die als Katalysator fungierenden Metallkomplexe können mit den beschriebenen chiralen Liganden ebenfalls einfach erzeugt werden. Die Liganden weisen eine große Substitutionsbreite auf, so dass die Katalysatoren auf bestimmte Eigenschaften, Funktionalitäten und Aktivitäten einfach, durch die Wahl entsprechender Substitutionsmuster eingestellt werden können. Weiterhin können so ebenfalls exzellent lösliche Katalysatorsysteme (z.B. in THF, Toluol, Benzol, CHCl₃, CH₂Cl₂, Alkoholen, Ketone, etc.) konstruiert werden, wodurch z.B. eine Diskriminierung von möglichen Substraten umgangen wird bzw. die Steuerung der Aktivitäten und Enantioselektivitäten über Lösungsmittel und Lösungsmittelgemische möglich ist. Die Liganden sind relativ unempfindlich gegenüber Luft (H₂O bzw. O₂) und die entsprechenden Katalysatoren besitzen eine exzellente Langzeitstabilität. Unterschiedliche Edukte für eine enantioselektive Umsetzung, wie z.B. Ketone für die asymmetrische Hydrierung, können auch selbst als Lösungsmittel für den Katalysator dienen, so dass auf zusätzliche Lösungsmittel verzichtet werden kann.

Ein weiterer Gegenstand der vorliegenden Erfindung ist die Verwendung der beschriebenen Katalysatoren zur enantioselektiven Hydrierung von Ketonen zur Herstellung entsprechender optisch aktiver Alkohole. Dabei werden bevorzugt Katalysatoren der allgemeinen Formeln **2-6** in Verbindung mit einem der beschriebenen Katalysator-Additive eingesetzt. Weiterhin ist Gegenstand der vorliegenden Erfindung ein enantioselektives Hydrierverfahren zur Herstellung von Alkoholen aus Ketonen.

### Kurze Beschreibung der Figuren:

Figur 1: Schematische Darstellung der Herstellung von chiralen NN(R)-Liganden **1** (R¹-R⁹ = Substituenten) aus bevorzugten Edukten.

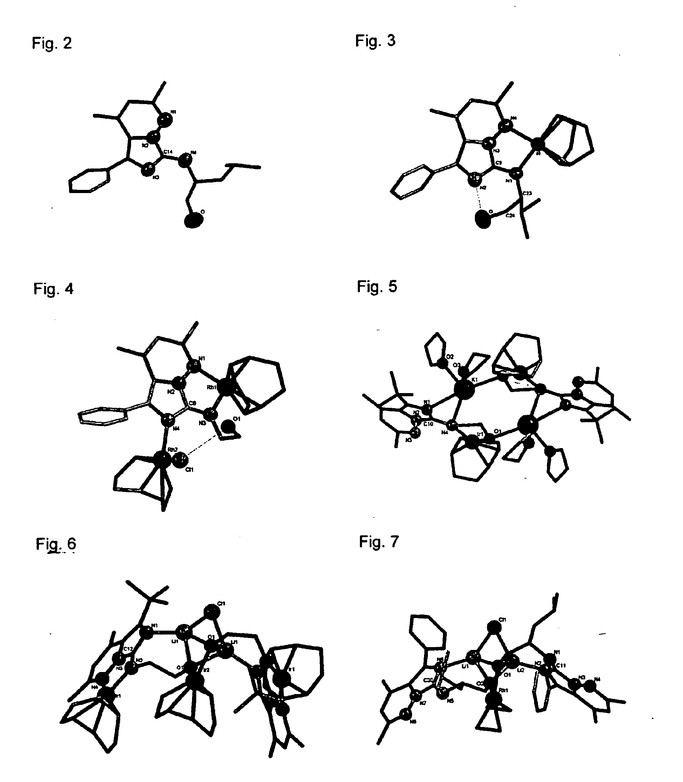

In Figur 2 ist beispielhaft die Molekülstruktur eines erfindungsgemäßen NN(R)-Liganden (**1a**) abgebildet. Die Struktur wurde mittels Röntgeneinkristallstrukturanalyse ermittelt. Ausgewählte Bindungslängen [Å] und -winkel [°]: N(1)-C(12) 1.305, N(1)-N(2) 1.373, N(2)-C(14) 1.365, N(2)-C(8) 1.414, N(3)-C(14) 1.327, N(4)-C(14) 1.361, C(9)-C(11) 1.355; C(14)-N(2)-N(1) 123.5, C(12)-N(1)-N(2) 114.0, C(14)-N(2)-C(8) 107.0, N(1)-N(2)-C(8) 128.5, C(14)-N(3)-C(7) 106.5, N(3)-C(14)-N(4) 127.9, N(4)-C(14)-N(2) 121.0.
In Figur 3 ist beispielhaft die Molekülstruktur eines erfindungsgemäßen Iridium-Komplexes (**2b**) abgebildet. Die Struktur wurde mittels Röntgeneinkristallstrukturanalyse ermittelt. Ausgewählte Bindungslängen [Å] und - winkel [°]: Ir-N1 2.071, Ir-N4 2.150, N3-N4 1.389, N3-C9 1.348, N1-C9 1.350, N2-C9 1.313, N2-O 2.771; N1-Ir-N4 80.4, C9-N1-Ir 112.3, N3-N4-Ir 106.5, N1-C9-N3 118.3, N2-C9-N1 131.4.
In Figur 4 ist beispielhaft die Molekülstruktur eines erfindungsgemäßen Rhodium-Komplexes **(3a)** abgebildet. Die Struktur wurde mittels Röntgeneinkristallstrukturanalyse ermittelt. Ausgewählte Bindungslängen [Å] und - winkel [°]: Rh1-N3 2.067, Rh1-N1 2.140, Rh2-N4 2.123, Rh2-Cl1 2.384, N1-N2 1.384; N3-Rh1-N1 81.2, N4-Rh2-Cl1 91.9, N2-N1-Rh1 106.2, C6-N2-N1 121.5, C6-N3-Rh1 111.8.
In Figur 5 ist beispielhaft die Molekülstruktur eines erfindungsgemäßen Iridium-Kalium-Komplexes **(4a)** abgebildet. Die Struktur wurde mittels Röntgeneinkristallstrukturanalyse ermittelt. Ausgewählte Bindungslängen [Å] und - winkel [°]: N1-K1 2.770(9), N2-N3 1.369(9), N4-Ir1 2.031(8), N4-K1 2.939(7), Ir1-01 1.990(6), O1-K1 2.664(7), O2-K1 2.628(9), O3-K1 2.667(8); Ir1-N4-K1 87.6(3), Ir1-O1-K1 133.4(3), O1-K1-N1 164.6(2), O1-K1-N4 115.6(2), N1-K1-N4 49.3(2), N4-Ir1-K1 57.0(2), O1-Ir1-K1 95.10(17).
In Figur 6 ist beispielhaft die Molekülstruktur eines erfindungsgemäßen Iridium-Lithium-Komplexes **(5a)** abgebildet. Die Struktur wurde mittels Röntgeneinkristallstrukturanalyse ermittelt. Ausgewählte Bindungslängen [Å] und - winkel [°]: I r1-N2 2.048, Ir1-N4 2.138, Ir2-O1 2.073, Li1-O1 2.013, Li1-N1 2.051, Li1-Cl1 2.345; O1-Ir2 -O1A 73.3, O1-U1-O1A 75.8, O1-Li1-N1 103.9, N2-Ir1-N4 80.5, Li1-Cl1-Li1A 62.2.
In Figur 7 ist beispielhaft die Molekülstruktur eines erfindungsgemäßen Rhodium-Lithium-Komplexes **(6a)** abgebildet. Die Struktur wurde mittels Röntgeneinkristallstrukturanalyse ermittelt. Ausgewählte Bindungslängen [Å] und - winkel [°]: N2-Li2 2.020(18), N3-N4 1.388(10), N6-Li1 2.032(15), O1-Li2 1.87(2), O1-Li1 1.992(17), O1-Rh1 2.040(7), O2-Li2 1.89(2), O2-Li1 2.025(17), O2-Rh1 2.054(7), Cl1-Li2 2.363(19), Cl1 Li1 2.395(17); O1-Li2-O2 82.6(10), O1-Li2-N2 105.0(9), OO2-Li2-N2 121.0(10), Li2-O1-Rh1 90.7(7), Li1-O1-Rh1 91.5(5), Li2-O2-Li1 71.1(7), O1-Rh1-O2 74.6(3).

### Ausführungsbeispiele:

### Allgemeines:

Alle Reaktionen und Katalysen mit luftempfindlichen Verbindungen wurden unter Schutzgas (Stickstoff bzw. Argon) bei Verwendung der Schlenktechnik und Handschuhboxen durchgeführt. Nichthalogenierte Lösungsmittel wurden über Natrium und Benzophenon und halogenierte Lösungsmittel über P₂O₅ unter Argon getrocknet und destilliert. Alle zur Synthese verwendeten Chemikalien sind kommerziell erhältlich und wurden ohne weitere Aufreinigungen verwendet. Die zur Katalyse verwendeten Substrate (prochirale Ketone) wurden zuvor über Molsieb und Argon getrocknet. Deuterierte NMR-Lösungsmittel wurden zuvor entgast, getrocknet, destilliert und in einer Handschuhbox gehandhabt.

Die NMR-Spektren wurden mit einem Bruker ARX 250 Spektrometer aufgenommen. Die chemische Verschiebung ist in ppm relativ zum deuterierten Lösungsmittel angegeben. Die Röntgeneinkristallstrukturanalysen wurden mit einem STOE-IPDS II, ausgerüstet mit einer Oxford Cryostream Tieftemperatur-Einheit, bestimmt. Die Strukturauflösungen und -verfeinerungen wurden mit SIR97 (A. Altomare, et al., J. Appl. Crystallogr. 1999, 32, 115-119), SHELXL-97 (G.M. Sheldrick, *SHELX-97,* Program for Crystal Structure Analysis (Release 97-2)., Institut für Anorganische Chemie der Universität, Göttingen, Germany, 1998) und WinGX (L.J. Farrugia, J. Appl. Crystallogr. 1999, 32, 837-838.) durchgeführt. Die Elementaranalysen wurden mit einem Vario elementar EL *III* Analysator bestimmt. Die Schmelzpunkte wurden in Glaskapillaren mit einem Stuart SMP3 Schmelzpunktapparat bestimmt. Die gaschromatographischen Untersuchungen wurden mit einem Agilent 6890N Gaschromatographen, ausgestattet mit einem 7683 Series Injector und Auto Sampler sowie einer Macherey-Nagel Lipodex-E Kapillarsäule (25 m x 0.25 mm) durchgeführt. Zur Bestimmung des Umsatzes wurde Dodecan als Standard verwendet. Die Katalysen/Druckexperimente wurden in Autoklaven N-MT5 (Multiple-Test-Reaktoren) von Parr Instrument durchgeführt.

### Ausführungsbeispiele zu Ligandensynthesen:

Die von uns in den Katalysatorsystemen eingesetzten Steuerliganden sind sehr einfach, ausgehend von sehr preiswerten chiralen Bausteinen, darzustellen.

### Beispiel 1a: 2-(2,4-Dimethyl-5-phenyl-imidazo[1,5-b]pyridazin-7-ylamino)-4-methyl-pentan-1-ol

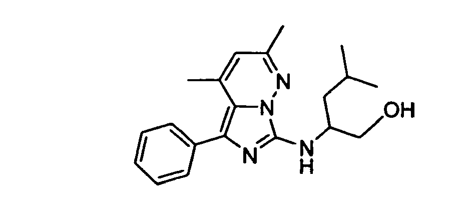

4.00 g (13 mmol) 2-Amino-5-methyl-3-phenacyl-1,3,4-oxadiazoliumbromid werden mit 3.35 mL (26 mmol, 3.04 g) S-(+)-Leucinol verrieben und kurz auf einer Heizplatte erhitzt. Es tritt eine heftige Reaktion ein begleitet von einem Farbwechsel von beige nach orange. Das auf RT abgekühlte, glasartige Produkt wird mit 15 mL Ethanol versetzt und kurz erwärmt. Das breiige Reaktionsgemisch wird mit 1.33 mL (13 mmol, 1.30 g) Acetylaceton versetzt, in Ethanol aufgeschlämmt und HCl hinzu gegeben. Das Reaktionsgemisch wird 4 h unter Rückfluss erhitzt. Die gelbe, fluoreszierende Lösung wird am Rotationsverdampfer eingeengt, der ölige Rückstand mit einigen ml Wasser aufgenommen. Anschließend erhitzt man die Lösung und filtriert den gebildeten Niederschlag ab. Das Filtrat wird bis zur basischen Reaktion mit 1 N NaOH versetzt, wobei es zur Bildung eines orangen klebrigen Produkts kommt. Danach wird das Wasser abdekantiert und der Rückstand in Ether aufgenommen. Nach Abziehen des Lösungsmittels und Trocknen am Vakuum erhält man ein oranges, kristallines Produkt. Ausbeute: 2.46 g (56 %), Schmp.: 57 °C. EA [%]: C₂₀H₂₆N₄O (338.43) gef.: C 70.97, H 7.74, N 16.55; ber.: C 70.80, H 7.72, N 16.46. ¹H-NMR (CDCl₃, δ[ppm]): 7.42-7.37(m, 2H, Hₒ, C₆H₅), 7.27-7.16(m, 3H, H_{m,p} C₆H₅), 5.81-5.80 (d, *J* = 1.1 Hz, 1H, H-3, Imidazopyridazin); 4.74-4.72(d, *J* = 5.4Hz, 1H, NH); 3.87-3.54(m, 3H, HN-C*H*-C*H₂*-OH); 2.19(s, 3H, CH₃-(C-2)); 2.07-2.06(d, *J* = 1.0Hz, 3H, CH₃-(C-4)); 1.73-1.59(m, 1H, C*H*-(CH₃)₂); 1.51-1.31(m, 2H, C*H₂*-CH(CH₃)₂); 0.87-0.82(t, *J* = 6.4Hz, 6H, (CH₃)₂). Gitterkonstanten: a = 11.222(2), b = 7.5421(15), c = 11.502(2) Å; β = 102.82(3)°, V = 949.2(3) Å³.

### Beispiel 1b: 2-(2,4-Dimethyl-5-phonyl-imidazo[1,6-b]pyridazin-7-ylamino)-3-methyl-butan-1-ol

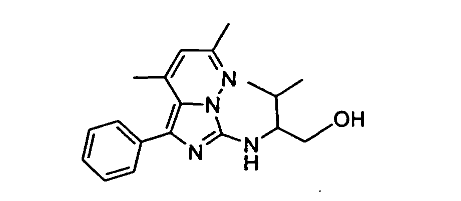

2.00 g (6.71 mmol) 2-Amino-5-methyl-3-phenacyl-1,3,4-oxadiazoliumbromid werden mit 1.38 g (13.42 mmol) L-Valinol bei RT verrieben und ca. 3 Minuten kräftig auf der Heizplatte erhitzt. Das auf RT abgekühlte glasartige Produkt wird in Ethanol gelöst, mit 0.59 mL (0.67 g, 6.71 mmol) Acetylaceton und 3 mL konz. HCl versetzt. Die neongelbe, fluoreszierende Reaktionslösung wird 3 h unter Rückfluss erhitzt und am Rotationsverdampfer eingeengt. Die zurückbleibende ölige Substanz wird in etwas Wasser gelöst. Anschließend erhitzt man die Lösung und filtriert den gebildeten Niederschlag ab. Das Filtrat wird bis zur basischen Reaktion mit 1 N NaOH versetzt, wobei es zur Bildung eines orangen, klebrigen Produktes kommt. Nach dem Absetzen des Produktes wird das Wasser abdekantiert, der Rückstand in Ether aufgenommen und in ein Schlenkgefäß gefüllt. Nach Abziehen des Lösungsmittels und Trocknen unter Vakuum erhält man ein oranges, kristallines Produkt. Ausbeute: 1.19 g (55 %), Schmp.: 43 °C. EA [%]: C₁₉H₂₄N₄O (324.40), gef.: C 70.04, H 7.45, N 17.12; ber.: C 70.34, H 7.46, N 17.27; ¹H-NMR (CDCl₃, δ[ppm]): 7.50-7.49(m, 2H, Hₒ, C₆H₅); 7.35-7.33(t, 2H, Hₘ, C₆H₅), 7.28-7.25(t, 1H, Hₚ, C₆H₅); 5.91-9.90(d, 1H, H-3, *J* = 1Hz, Imidazopyridazin); 4.98-4.97 (d, 1H, NH, *J* = 6Hz); 3.88-3.78(m, 2H, C*H*₂-OH); 3.61-3.59(m, 1H, C*H*-NH); 2.29 (s, 3H, CH₃-(C-2)); 2.16-2.15(d, 3H, *J* = 1Hz, CH₃-(C-4)); 2.11-2.10(m, 1H, C*H-*(CH₃)₂); 1.04-1.03(d, 3H, CH₃); 1.02-1.01(d, 3H, CH₃).

### Beispiel 1c: 2-(2,4-Dimethyl-5-phenyl-imidazo[1,5-b]pyridazin-7-ylamino)-butan-1-ol

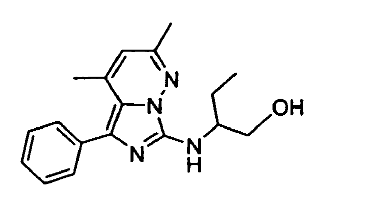

2.00 g (6.71 mmol) 2-Amino-5-methyl-3-phenacyl-1,3,4-oxadiazoliumbromid werden mit 1.27 mL (1.20 g; 13.42 mmol) (S)-(+)-2-Amino-1-butanol bei RT verrieben. Anschließend wird das Reaktionsgemisch kurz aber kräftig (- 1 min) auf der Heizplatte erhitzt bis alles klar schmilzt. Nach dem Abkühlen auf RT wird das glasartige/spröde Produkt in 20 mL Ethanol unter leichter Erwärmung gelöst, mit 0.69 mL (0.67 g; 6.71 mmol) Acetylaceton und 2 mL konz. HCl versetzt. Die neongelbe Reaktionslösung wird 2 Stunden unter Rückfluss erhitzt, am Rotationsverdampfer eingeengt und dann in einigen mL Wasser gelöst. Zu dieser Lösung tropft man 1 N NaOH bis zur basischen Reaktion. Nach dem Absetzen eines orange/roten Produktes wird abgesaugt, mehrmals mit Wasser gewaschen, das Produkt danach in Ether gelöst und filtriert. Nach dem Abziehen des Ethers wird im Vakuum getrocknet und man erhält ein oranges/rotes Produkt. Ausbeute: 1.29 g (62 %). EA [%]: C₁₈H₂₂N₄O (310.39), gef.: C 68.75, H 7.09, N 17.60; ber.: C 69.65, H 7.14, N 18.05. ¹H-NMR (CDCl₃, δ[ppm]): 7.45-7.41(dd, 2H, Hₒ, C₆H₅₎, 7.29-7.17(m, 3H, H_{m,p} C₆H₅), 5.84-5.83 (d, 1H, H-3, *J* = 1 Hz, Imidazopyridazin); 5.32(sbs, 1H, OH); 4.85-4.83(d, 1H, NH); 3.79-3.60(m, 3H, CH-CH₂-OH); 2.22(s, 3H, CH₃-(C-2)); 2.09-2.08(d, 3H, *J* = 1 Hz, CH₃-(C-4)); 1.72-1.53(m, 2H, CH₂-CH₃); 0.98-0.92(t, 3H, CH₂-CH₃).

### Beispiel 1d: 2-(2,4-Dimethyl-5-phenyl-imidazo[1,5-b]pyridazin-7-ylamino)-2-phenyl-methanol

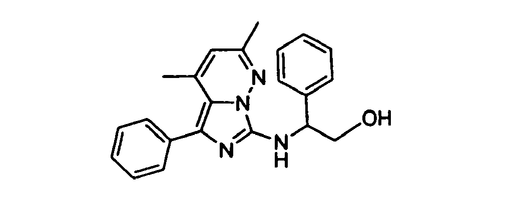

2.00 g (6.70 mmol) 2-Amino-5-methyl-3-phenacyl-1,3,4-oxadiazoliumbromid werden bei RT mit 1.80 g (13 mmol) S-(+)-Phenylglycinol verrieben und ca. 1 Minute kräftig auf einer Heizplatte erhitzt. Das auf RT abgekühlte, glasartige Produkt wird in Ethanol gelöst, mit 0.69 mL (1.34 g, 6.70 mmol) Acetylaceton sowie 3 mL konz. HCl versetzt. Anschließend wird die neongelbe Reaktionslösung 4 h unter Rückfluss erhitzt und am Rotationsverdampfer eingeengt. Die zurückbleibende ölige Substanz wird mit Wasser aufgenommen und bis zur basischen Reaktion mit 1 N NaOH versetzt. Dabei kommt es zur Bildung eines roten klebrigen Produkts. Das Wasser wird abdekantiert, der Rückstand in Ether aufgenommen und filtriert. Nach Abziehen des Lösungsmittels und Trocknen am HV wird mit Ethanol umkristallisiert. Man erhält ein gelbes, kristallines Pulver. Ausbeute: 1.20 g (50 %); Schmp.: 58 °C. EA [%]: C₂₂H₂₂N₄O x ½ H₂O (367.44) gef.: C 71.19, H 6.17, N 14.86, ber.: C 71.91, H 6.31, N 15.25. ¹H-NMR (CDCl₃, δ[ppm]): 7.46-7.23(m, 10H, 2 x C₆H₅), 5.87-5.86(d, 1H, H-3, Imidazopyridazin), 5.22-5.20(d, 1H, NH, *J* = 6Hz), 4.93-4.87(m, 1H, C*H*-NH), 4.04-3.81(m, 2H, C*H*₂-OH), 2.19(s, 3H, CH₃-(C-2)), 2.11(s, 3H, CH₃-(C-4)).

### Beispiel 1e: 2-(5-tert-Butyl-2,4-dimethyl-imidazo[1,5-b]pyridazin-7-ylamino)-4-methyl-pentan-1-ol ethanol-addukt

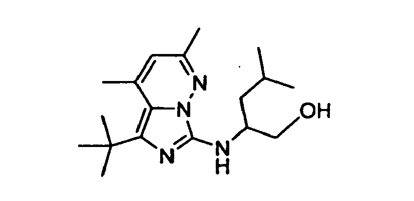

3.00 g (10.70 mmol) 2-Amino-5-methyl-3-(2-oxo-3,3-dimethylbutyl)-1,3,4-oxadiazoliumbromid werden mit 2.50 g (21.40 mmol) S-(+)-Leucinol verrieben und kurz erhitzt. Das auf RT abgekühlte glasartige Produkt wird mit Ethanol versetzt und kurz erwärmt bis eine klare Lösung entsteht. Dazu werden 1.09 mL (1.07 g, 10.70 mmol) Acetylaceton und 3 mL HCl gegeben. Anschließend wird die neongelbe, fluoreszierende Reaktionslösung 4 h unter Rückfluss erhitzt und am Rotationsverdampfer eingeengt. Der Rückstand wird mit H₂O aufgenommen und mit 1 N NaOH bis zur basischen Reaktion (pH = 9) versetzt, wobei es zur Bildung eines orangen, glasartigen Niederschlages kommt. Nach dem Absetzen des Produktes wird das Wasser abdekantiert, der Rückstand in Ether aufgenommen und über Kieselgel (2x) filtriert. Nach Abziehen des Lösungsmittels und Trocknen unter Vakuum erhält man ein oranges, klebriges Produkt. Ausbeute: 1.83 g (54 %). EA [%]: C₁₈H₂₉N₄O * 0.5 C₂H₅OH (344.44) gef.: C 67.68, H 10.08, N 16.82, ber.: C 67.03, H 9.47, N 16.46. ¹H-NMR (CDCl₃, δ[ppm]): 5.83(s, 1H, H-3, Imidazopyridazin); 4.82-4.80(d, 1H, NH); 3.83-3.67(m, 3H, NH-C*H*-C*H*₂-OH); 2.51(s, 3H, CH₃-(C-4)); 2.23(s, 3H, CH₃-(C-2)); 1.76-1.68(m, 1H, C*H*-(CH₃)₂); 1.52-1.45(m, 2H, C*H₂*-CH-(CH₃)₂); 1.41(s, 9H, C(C*H*₃)₃); 0.96-0.90(t, 6H, (CH₃)₂).

### Beispiel 1f: 4-Methyl-2-(2,4,5-triphenyl-imidazo[1,5-b]pyridazin-7-ylamino)-pentan-1-ol ethanol-addukt

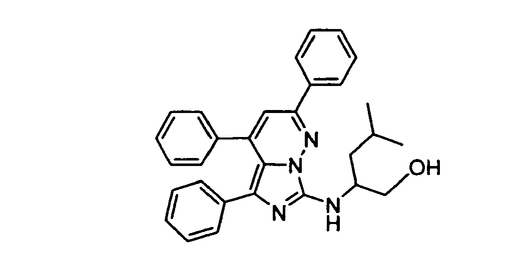

4.00 g (13.40 mmol) 2-Amino-5-methyl-3-phenylacyl-1,3,4-oxadiazolimbromid werden mit 3.48 mL (26.80 mmol, 3.04 g) S-(+)-Leucinol verrieben und kurz auf einer Heizplatte erhitzt. Das auf RT abgekühlte, glasartige Produkt wird in 15 mL Ethanol gelöst, mit 2.90 g (13.40 mmol) Dibenzoylmethan und 3 mL HCl versetzt. Anschließend wird 2 h unter Rückfluss erhitzt und die rote Reaktionslösung am Rotationsverdampfer eingeengt. Die zurückbleibende schleimige Substanz wird mehrmals mit Wasser gewaschen bis das Produkt fest wird. Anschließend erhitzt man die Lösung für einige Minuten unter Rühren in Ethanol/ Wasser (2:1) und versetzt bis zur basischen Reaktion mit 1 N NaOH, wobei es zur Bildung eines dunkelroten Niederschlages kommt. Dieser wird mehrfach mit Wasser gewaschen. Der Rückstand wird aus Ethanol umkristallisiert. Man erhält ein rotes kristallines Produkt. Ausbeute: 2.40 g (38 %). Schmp.: 162 °C. EA [%]: C₃₀H₃₀N₄O x 2 C₂H₅OH (554.71). gef.: C 73.38, H 7.26, N 10.66, ber.: C 73.61, H 7.63, N 10.10. ¹H-NMR (CDCl₃, δ[ppm]): 7.88-6.90(m, 15H, 3 x C₆H₅); 6.59(s, 1H, H-3, Imidazopyridazin); 5.62(s, 1H, CH₂-O*H*): 5.02(d, 1H, CH-N*H*); 3.95-3.57(m, 5H, NH-C*H*-C*H₂*-OH / C*H₂*, Ethanol); 1.76-1.47(m, 3H, C*H₂*-C*H*-(CH₃)₂); 1.17-1.11(t, 3H, CH₃, Ethanol); 0.94-0.89(t, 6H, CH-(C*H₃*)₂).

### Beispiel 1g: 2-(2,4-Dimethyl-5-phenyl-imidazo[1,5-b]pyridazin-7-ylamino)-3-phenyl-propan-1-ol semihydrat

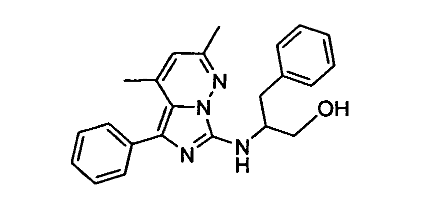

2.73 g (9.16 mmol) 2-Amino-5-methyl-3-phenacyl-1,3,4-oxadiazoliumbromid werden mit 2.77 g (18.33 mmol) L-Phenylalaninol bei RT verrieben. Anschließend wird das Reaktionsgemisch kräftig (- 5 min) auf der Heizplatte erhitzt. Nach dem Abkühlen auf RT wird das glasartige beige Produkt in 25 mL Ethanol gelöst und man gibt 0.94 mL (0.92 g; 9.16 mmol) Acetylaceton und 2 mL konz. HCl hinzu. Die orange/neongelbe Reaktionslösung wird 2 Stunden unter Rückfluss erhitzt, eingeengt und dann in einigen mL Wasser gelöst. Zu dieser Lösung tropft man 1 N NaOH bis zur basischen Reaktion. Nach dem Absetzen eines roten (schleimigen) Produktes wird mehrmals mit Wasser gewaschen und aus Ethanol umkristallisiert. Nach dem Trocknen im Vakuum erhält man einen orangen Feststoff. Ausbeute: 2.10 g (62 %). Schmp.: 56 °C. EA [%]: C₂₃H₂₅N₄O_{1.5} (381.44), gef. C 72.31, H 6.81, N 14.82, ber. C 72.42, H 6.61, N 14.69. ¹H-NMR (CDCl₃, δ[ppm]): 7.44-7.08(m, 10H, C₆H₅); 5.83-5.82 (d, 1H, H-3, *J* = 1 Hz, Imidazopyridazin); 4.95-4.92(d, 1H, NH); 3.98-3.95(m, 1H, C*H*-NH); 3.84-3.65(m, 2H, C*H₂*-OH); 2.96-2.88(q, 2H, C*H₂*-C₆H₅); 2.17(s, 3H, C*H₃*-(C-2)); 2.09-2.08(d, 3H, *J* = 1Hz, C*H*₃-(C-4)).

### Beispiel 1h: 3-(2,4-Dimethyl-5-phenyl-imidazo[1,5-b]pyridazin-7-ylamino)-propan-1,2-diol

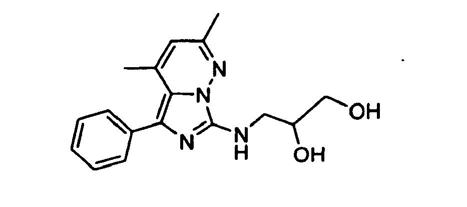

2.00 g (6.71 mmol) 2-Amino-5-methyl-3-phenacyl-1,3,4-oxadiazoliumbromid werden mit 1.22 g (13.4 mmol) (R)-3-Amino-1,2-propandiol bei RT verrieben. Anschließend wird das Reaktionsgemisch kurz auf der Heizplatte erhitzt, wobei eine exotherme Reaktion zu beobachten ist. Nach dem Abkühlen auf RT wird das Produkt in ca. 20 mL Ethanol gelöst. Danach gibt man 0.69 mL (0.67 g, 6.71 mmol) Acetylaceton und 1.50 mL konz. HCl hinzu. Die gelbe Reaktionslösung wird 2 h unter Rückfluss erhitzt, am Rotationsverdampfer eingeengt und dann die rote, zähe Flüssigkeit in einigen mL Wasser gelöst. Zu dieser Lösung tropft man 1 N NaOH bis zur basischen Reaktion. Nach dem Absetzen eines roten Produkts wird die überstehende Lösung abdekantiert, das Produkt in Ether gelöst und filtriert. Nach dem Abziehen des Ethers wird im Vakuum getrocknet und man erhält ein oranges Produkt. Ausbeute: 1.02 g (49 %), Schmp.: 65.8 °C; ¹H-NMR (C₆D₆, δ[ppm]): 7.61-7.58(m, 2H, Hₒ, C₆H₅); 7.23-7.07(m, 3H, H_{m,p}, C₆H₅), 5.22-5.22(d, *J*=0.9 Hz, 1H, H-3, Imidazopyridazin); 5.21-5.17(t, *J*=6.3 Hz, 2H, N*H*-CH₂); 3.87-3.82(qui, *J*=5.1 Hz, 1H, C*H*-CH₂-OH); 3.76-3.68(m, 2H, CH-C*H₂*-OH); 3.63-3.55(q, *J*=5.4 Hz, 2H, NH-C*H₂*-CH-OH); 1.92(s, 3H, CH₃-(C-2)); 1.77-1.76(d, *J*=0.9 Hz, 3H, CH₃-(C-4)).

### Beispiel 1i: Essigsäure-2-(2,4-dimethyl-5-phenyl-imidazo[1,5-b]pyridazin-7-ylamino)-4-methyl-pentyl-ester

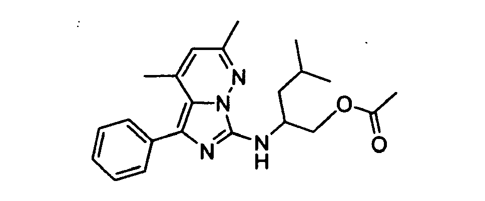

1.46 g (4.31 mmol) **1a** werden in 15 mL Eisessig 2.5 Stunden refluxiert. Anschließend wird die dunkelrote Reaktionslösung langsam eingeengt und das Produkt mit Wasser versetzt. Das schleimige Produkt wird mehrfach mit Wasser gewaschen in Ether gelöst und anschließend filtriert. Nach dem Abziehen des Ethers wird im Vakuum getrocknet und man erhält ein zähes rotes Produkt. Ausbeute: 1.21 g (74 %), Schmp.: schleimig/zäh bei RT; ¹H-NMR (DMSO-d₆, δ[ppm]): 7.52-7.50(m, 2H, Hₒ C₆H₅); 7.40-7.26(m, 3H, H_{m,p} C₆H₅); 6.06(s, 1H, H-3, Imidazopyridazin); 5.93-5.90(d, *J*=9.3 Hz, 1H, NH); 4.31-4.20(m, 1H, NH-C*H*-CH₂-O); 4.18-4.05(m, 2H, C*H₂*-O); 2.29(s, 3H, CH₃); 2.14(s, 3H, CH₃); 1.91(s, 3H, CH₃); 1.78-1.61(m, 2H, C*H₂*-CH(CH₃)₂); 1.36-1.29(m, 1H, C*H*-(CH₃)₂); 0.91-0.89(m, 6H, (CH₃)₂).

### Ausführungsbeispiele zur Komplexsynthese:

Aus den effizient herzustellenden Liganden können die als Hauptkomponente des Katalysatorsystems wirkenden Metallkomplexe (hier das Beispiel Iridium und Rhodium, jedoch erweiterbar auf alle Metalle und eine Vielzahl von Elementen des Periodensystems der Elemente (PSE)) synthetisiert werden.

### Beispiel 2a: 2-(2,4-Dimethyl-5-phenyl-imidazo[1,5-b]pyridazin-7-ylamido)-4-methyl-pentan-1-ol-iridium-cyclooctadien-komplex

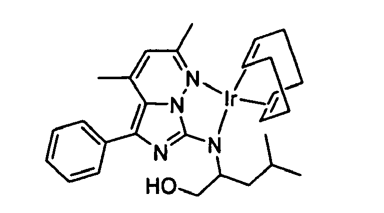

0.50 g (1.47 mmol) **1a** gelöst in 40 mL THF werden bei -78 °C tropfenweise mit 0.875 mL (1.47 mmol) *n*-BuLi (1.6 M in *n*-Hexan) versetzt. Die dunkelorange Reaktionslösung wird eine halbe Stunde bei -78 °C gerührt und anschließend lässt man auf RT erwärmen. Danach hebert man eine Lösung von 0.47 g (0.70 mmol) Chlor-1,5-cyclooctadien-iridium (I), dimer in 15 mL THF hinzu, wobei ein Farbumschlag nach dunkelgrün eintritt. Die Reaktionslösung wird kurz erhitzt, 16 h bei RT gerührt und anschließend das Lösungsmittel abgezogen. Das auf 5 mL eingeengte Reaktionsgemisch wird mit Ether versetzt und filtriert. Aus dem Filtrat erhält man bei -30 °C ein grünes, kristallines Produkt. Ausbeute: 0.73 g (84 %); Schmp.: 98.4 °C. EA [%]: C₂₈H₃₇IrN₄O (637.81) gef.: C 52.45, H 6.15, N 8.21; ber.: C 52.68, H 5.8, N 8.7. ¹H-NMR (THF-d₈, δ[ppm]): 7.56-7.25(m, 5H, C₆H₅); 5.88 (s,1H, H-3, Imidazopyridazin); 4.36-3.55(m, 7H, C*H₂*-OH /4 x C*H*, COD/C*H*-N); 2.36(s, 3H, CH₃-(C-2)); 2.23(s, 3H, CH₃-(C-4); 2.19-2.11(m, 6H, C*H₂*-CH₃/2 x C*H*_{*2*,} COD); 1.76-1.63(m, 5H, C*H*-(CH₃)₂, 2 x C*H₂*, COD); 0.91-0.87(q, 6H, CH-(C*H₃*)₂).

### Beispiel 2b: 2-(2,4-Dimethyl-5-phonyl-imidazo[1,5-b]pyridazin-7-ylamido)-3-methyl-butan-1-ol-iridium-cyclooctadien-komplex

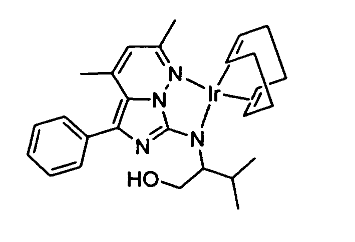

0.95 g (2.93 mmol) **1b** gelöst in 10 mL THF werden bei -78 °C tropfenweise mit 1.83 mL (2.93 mmol) *n*-BuLi (1.6 M in *n*-Hexan) versetzt. Die dunkelorange Reaktionslösung wird eine weiter halbe Stunde bei -78 °C gerührt. Anschließend lässt man auf RT erwärmen und hebert dann eine Lösung von 0.98 g (1.46 mmol) Chlor-1,5-cyclooctadien-iridium (I), dimer in 15 mL THF hinzu, wobei ein Farbumschlag nach tiefblau/türkis eintritt. Die Reaktionslösung wird kurz erhitzt, 16 h bei RT gerührt und anschließend das Lösungsmittel abgezogen. Das auf 5 mL eingeengte Reaktionsgemisch wird mit Ether versetzt. Der darin lösliche Komplex wird mittels einer Schlauchfritte filtriert. Aus dem Filtrat erhält man bei - 30 °C ein blau-schwarzes, kristallines Produkt. Ausbeute: 1.43 g (78 %); Schmp.: 188.4 °C. ¹H-NMR (CDCl₃, δ[ppml): 7.48-7.26(m, 5H, C₆H₅); 6.58-6.54(d, 1H, OH); 5.69-5.68(d, 1H, *J*=1Hz, H-3, Imidazopyridazin); 4.35-3.77(m, 5H, C*H₂*-OH /2 x C*H*, COD/C*H*-N); 2.95-2.91(m, 1H, CH, COD); 2.78-2.76(m, 1H, CH, COD); 2.34(s, 3H, CH₃-(C-2)); 2.19-2.18(d, 3H, *J*=1Hz, CH₃-(C-4)); 2.25-2.13(m, 5H, 2 x C*H₂*, COD/C*H*-(CH₃)₂); 1.80-1.58(m, 4H, 2 x C*H₂*,COD); 1.02-0.99(d, 3H, CH₃); 0.88-0.85(d, 3H, CH₃). Gitterkonstanten: a = 11.131(2), b = 10.160(2), c = 11.208(2) Å; β = 102.97(3)°, V = 1235.2(4) Å³.

### Beispiel 2c: 2-(2,4-Dimethyl-5-phenyl-imidazo[1,5-b]pyridazin-7-ylamino)-butan-1-ol-iridium-cyclooctadien-komplex

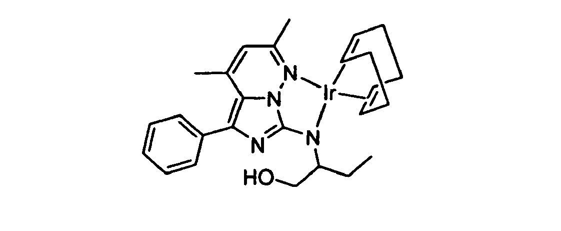

0.55 g (1.77 mmol) **1c** werden in 10 mL THF gelöst und bei -78 °C tropfenweise mit 1.11 mL (1.77 mmol) *n*-BuLi (1.6 M in *n*-Hexan) versetzt. Die resultierende dunkelorange Reaktionslösung wird eine weitere halbe Stunde bei -78 °C gerührt und anschließend lässt man langsam auf RT erwärmen. Dann hebert man eine Lösung von 0.59 g (0.89 mmol) Chlor-1,5-cyclooctadien-iridium (I), dimer in 10 mL THF hinzu, wobei ein Farbumschlag nach türkis erfolgt. Die Reaktionslösung wird mehrmals kurz erhitzt und dann 16 h bei RT gerührt. Anschließend engt man bis auf 5 mL ein. Nach einer Zugabe von 20 mL Ether wird kurz erwärmt und dann filtriert. Aus dem Filtrat erhält man bereits nach kurzer Zeit bei -30 °C ein blau-schwarzes Produkt, welches im Vakuum getrocknet wird. Ausbeute: 0.66 g (61 %); Schmp.: 286 °C. ¹H-NMR (CD₂Cl₂, δ[ppm]): 7.55-7.30(m, 5H, C₆H₅); 6.56-6.54(d, 1H, OH); 5.81(s, 1H, H-3, Imidazopyridazin); 4.38-3.41(m, 7H, C*H₂*-OH /4 x C*H*, COD/C*H*-N); 2.40(s, 3H, CH₃-(C-2)); 2.26(s, 3H, CH₃-(C-4)); 2.31-2.15(m, 4H, 2 x C*H₂*, COD); 1.80-1.66(m, 6H, C*H₂*-CH₃/2 x C*H*_{*2*,} COD); 0.99-0.94(t, 3H, CH₂-C*H₃*).

### Beispiel 2d: 2-(2,4-Dimethyl-5-phenyl-imidazo[1,5-b]pyridazin-7-ylamido)-3-phenyl-propan-1-ol-iridium-cyclooctadien-komplex

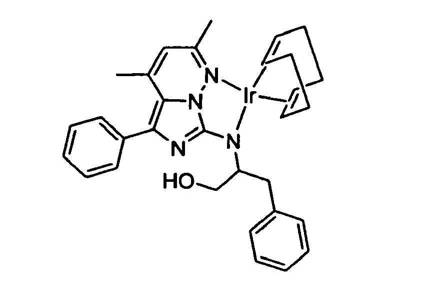

Eine Lösung von 0.40 g (1.08 mmol) **1g** in 15 mL THF werden bei -78 °C tropfenweise mit 0.675 mL (1.08 mmol) *n*-BuLi (1.6 M in Hexan) versetzt. Die dunkelbraune Lösung wird eine halbe Stunde bei -78 °C gerührt und anschließend lässt man auf RT erwärmen. Dann hebert man 0.35 g (0.54 mmol) Chlor-1,5-cyclooctadien-iridium(I) dimer in THF hinzu, wobei ein sofortiger Farbumschlag nach dunkelblau/türkis eintritt. Die Reaktionslösung wird einmal kurz erhitzt und dann 16 h bei RT gerührt. Anschließend engt man bis zur Trockne ein, löst den Komplex in Toluol und filtriert die Lösung. Aus dem Filtrat erhält man bei -30 °C ein dunkelgrünes kristallines Produkt. Ausbeute: 0.21 g (29 %); ¹H-NMR (CDCl₃, δ [ppm]): 7.54-7.09(m, 10H, 2 x C₆H₅); 5.78(s, 1 H, H-3, Imidazopyridazin); 4.28-4.21(m, 2H, C*H₂*-OH): 4.21-3.64(m, 1H, C*H*-NH); 3.43-3.06(m, 4H, 4 x CH, COD); 2.33(s, 3H, C*H₃*-(C-2)); 2.21(s, 3H, C*H₃*-(C-4)); 2.09-1.56(m, 10H, C*H₂*-C₆H₅, 4 x C*H₂*, COD).

### Beispiel 3a:

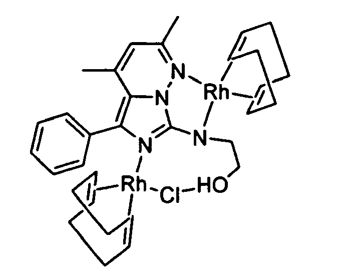

0.92 g (3.25 mmol) 2-(2,4-Dimethyl-5-phenyl-imidazo[1,5-*b*]pyridazin-7-ylamino)-ethanol werden in 30 mL THF gelöst und bei -70 °C mit 2.03 mL (3.25 mmol) *n-*BuLi (1.6 M in *n*-Hexan) versetzt. Nach 30 Minuten rühren bei -70 °C lässt man langsam auf RT erwärmen. Bei RT wird eine Suspension von 1.60 g (3.25 mmol) Chlor-1,5-cyclooctadien-rhodium (I), dimer in 15 mL THF zugegeben. Die dunkelgrüne Reaktionslösung wird mehrfach kurz erwärmt und 16 Stunden gerührt. Nach dem Entfernen des Lösungsmittels im Vakuum gibt man 40 mL Toluol hinzu und filtriert. Aus dem Filtrat erhält man -30 °C dunkelrote Kristalle. Ausbeute: 0.73 g (30 %), Schmp.: 212 °C; C₃₂H₄₁ClN₄ORh₂ (738.96); Kristallographische Daten: Kristallfarbe/-habitus: dunkelrote Nadeln; Kristallgröße: 0.40x0.02x0.01 mm³; Kristallsystem: monoklin; Raumgruppe: P2(1)/c; Gitterkonstanten: a=12.0452(6), b=16.6832(14), c=15.5336(9) Å; β=107.402(6)°, V=2978.6(3) Å³; Formeleinheit: Z=4; ρ_{ber}=1.648 Mg/cm³; 5442 Reflexe gemessen, 2452 symmetrieunabhängige Reflexe, [I>2sigma(I)], R=0.0648, wR²(alle Daten)=0.1398, 357 Parameter.

### Beispiel 3b:

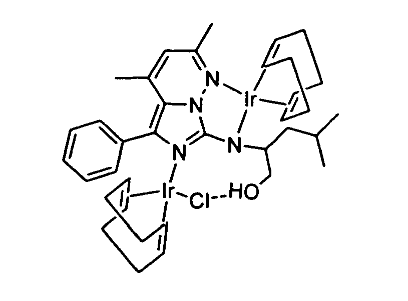

0.60 g (1.77 mmol) **1a** gelöst in 25 mL THF werden bei -70 °C tropfenweise mit 1.10 mL (1.77 mmol) *n*-BuLi (1.6 M in *n*-Hexan) versetzt. Die Reaktionslösung wird eine weitere halbe Stunde bei -70° C gerührt und man lässt dann langsam auf RT erwärmen. Anschließend hebert man eine Lösung von 1.19 g (1.77 mmol) Chlor-1,5-cyclooctadien-iridium (I), dimer in 15 mL THF hinzu. Eine sofortige Farbänderung von orange nach blau tritt ein. Das Reaktionsgemisch wird kurz erhitzt, über Nacht gerührt und dann das Lösungsmittel abgezogen. Anschließend wird der Rückstand mit Toluol extrahiert. Das Filtrat wird bis zur Trockene eingeengt und 2-mal mit 15 mL Hexan gewaschen. Man erhält ein blau-schwarzes kristallines Produkt. Ausbeute: 1.13 g (66 %); EA [%]: C₃₆H₄₉Cllr₂N₄O ^{*} C₄H₈O (1046.20), ber. C 45.88, H 5.44, N 5.35, gef. C 45.51, H 5.64, N 5.80. ¹H-NMR (THF-d₈, δ[ppm]): 7.46-7.17(m, 5H, C₆H₅); 5.78-5.77(d, 1H, H-3, Imidazopyridazin); 4.28-3.45(m, 11H, C*H₂*-OH /8 x C*H*, COD/ C*H*-N); 2.29(s, 3H, CH₃-(C-2)); 2.15(s, .. 3H, CH₃-(C-4); 1.99-1.16(m, 19H, C*H₂*-CH₃, C*H*-(CH₃)₂, 8 x C*H₂*(COD)); 0.79-0.78, 0.76-0.75(dd, 6H, CH-(C*H₃*)₂).

### Beispiel 4a:

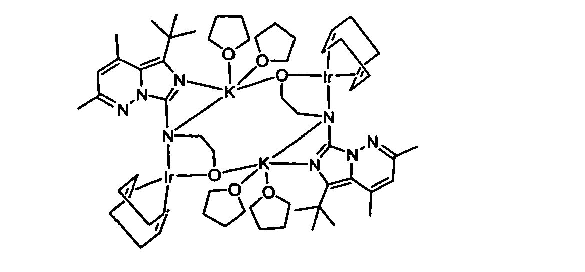

0.10 g (0.18 mmol) 2-(5-*tert*-Butyl-2,4-dimethyl-imidazo[1,5-*b*]pyridazin-7-ylamido)-ethanol-iridium-cyclooctadien gelöst in 5 mL THF werden bei RT mit 0.035 g (0.18 mmol) K[N(SiMe₃)₂] versetzt und kurz gerührt. Dabei ist eine Farbänderung von blau nach rot- orange zu beobachten. Nach etwa 2 h tritt Kristallisation ein. Anschließend filtriert man von dem roten, kristallinen Produkt ab. Ausbeute: 0.055 g (20 %); C₆₀H₉₆Ir₂K₂N₈O₆ (1488.04); Kristallographische Daten: Kristallfarbe/- habitus: rote Nadeln; Kristallgröße: 0.23x0.09x0.08 mm³; Kristallsystem: monoklin; Raumgruppe: P2(1)/n; Gitterkonstanten: a=11.131(8), b=21.219(17), c=13.429(11) Å; β=93.105(6)°, V=3167.1(4) Å³; Formeleinheit: Z=2; ρ_{ber}=1.560 Mg/cm³; 6016 Reflexe gemessen, 2763 symmetrieunabhängige Reflexe, [I>2sigma(I)], R=0.0443, wR²(alle Daten)=0.0796, 389 Parameter.

### Beispiel 5a:

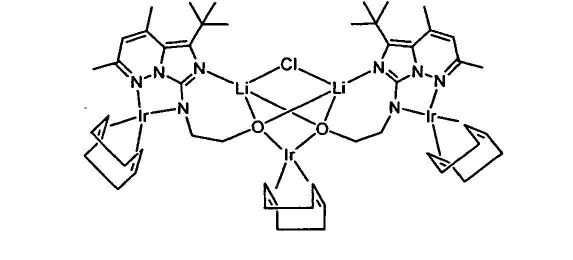

Eine Lösung von 0.64 g (2.44 mmol) 2-(5-*tert*-Butyl-2,4-dimethyl-imidazo[1,5-*b*]pyridazin-7-ylamino)-ethanol in 25 mL THF werden bei -70 °C tropfenweise mit 3.05 mL (4.88 mmol) *n*-BuLi (1.6 M in *n*-Hexan) versetzt. Die violette Reaktionslösung wird eine weitere halbe Stunde bei -70 °C gerührt und anschließend lässt man langsam auf RT erwärmen. Bei RT hebert man eine warme Lösung von 0.82 g (1.22 mmol) Chlor-1,5-cyclooctadien-iridium (I), dimer in 15 mL THF hinzu und ein sofortiger Farbumschlag nach dunkelgrün tritt ein. Die Reaktionslösung wird kurz gerührt und dann bei RT über Nacht ruhig stehengelassen (nach kurzer Zeit setzt Kristallisation ein). Anschließend filtriert man von dem blau-schwarzen kristallinen Produkt ab und wäscht einmal mit Ether. EA [%]: C₅₂H₇₆Cllr₃Li₂N₈O₂ * 2 C₄H₈O (1615.34), ber.: C 44.61, H 5.74, N 6.94, gef.: C 45.00, H 6.00, N 6.82; ¹H-NMR (CD₂Cl₂, δ[ppm]): 5.73(s, 2H, 2 x H-3, Imidazopyridazin); 4.47-4.42(t, 2H, C*H₂*-OH); 4.39-4.37(t, 2H, C*H₂*-OH); 4.08-4.03(d, 4H, 2 x C*H₂*-N); 3.98-3.08(m, 12H, 12 x CH, COD); 2.58(s, 6H, 2 x CH₃-(C-2)); 2.35(s, 6H, 2 x CH₃-(C-4); 2.17-1.76(m, 24 H, 12 x CH₂, COD); 1.55(s,18H,2 x C-(C*H₃*)₃). Gitterkonstanten: a = 15.1505(12), b = 18.1612(6), c = 26.4661(12) Å; β = 92.991(8)°, V = 7272.3(7) Å³.

### Beispiel 5b:

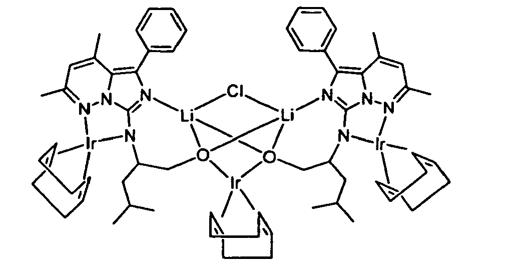

0.70 g (2.06 mmol) **1a** gelöst in 25 ml THF werden bei -70 °C tropfenweise mit 2.58 mL (4.12 mmol) *n*-BuLi (1.6 M in *n*-Hexan) versetzt. Dabei ist ein Farbumschlag von orange nach violett zu beobachten. Die Reaktionslösung wird eine weitere halbe Stunde bei -70 °C gerührt und man lässt dann langsam auf RT erwärmen. Anschließend hebert man eine warme Lösung von 1.04 g (1.55 mmol) Chlor-1,5-cyclooctadien-iridium (I), dimer in 15 mL THF hinzu. Ein sofortige Farbänderung nach dunkelblau tritt ein. Die Reaktionslösung wird kurz gerührt, über Nacht ruhig stehengelassen und dann das Lösungsmittel abgezogen. Das blau-schwarze Produkt wird anschließend in 20 mL Toluol gelöst und filtriert. Nach einiger Zeit setzt Kristallisation ein. Ausbeute: 1.86 g (56 %), EA [%]: C₆₄H₈₄Cllr₃Li₂N₈O₂ * C₄H₈O (1696.67) ber.: C 48.09, H 5.42, N 6.60, gef.: C 48.16, H 5.74, N 6.44; ¹H-NMR (CD₂Cl₂, δ[ppm]): 7.65-7.18(m, 10H, 2 x C₆H₅); 5.80-5.78(d, 2H, 2 x H-3, Imidazopyridazin); 4.41-3.74(m, ,18H, 2 x C*H₂*-OH /12 x C*H*, COD/2 x CH-N); 2.40(s, 6H, 2 x CH₃-(C-2)); 2.31-2.25(d, 6H, 2 x CH₃-(C-4); 2.09-0.9.6(m, 30H, 2 x C*H₂*-CH₃, 2 x C*H*-(CH₃)₂, 12 x C*H₂*, COD); 0.96-0.94, 0.83-0.80(dd,12H, 2 x CH-(C*H₃*)₂).

### Beispiel 6a:

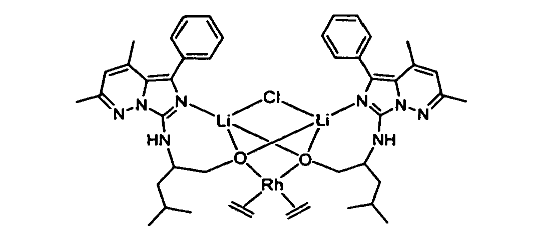

0.60 g (1.77 mmol) **1a** gelöst in 25 mL THF werden bei -70 °C tropfenweise mit 1.10 mL (1.77 mmol) *n*-BuLi (1.6 M in *n*-Hexan) versetzt. Die Reaktionslösung wird eine weitere halbe Stunde bei -70 °C gerührt und dann langsam auf RT erwärmt. Anschließend hebert man bei -30 °C eine Lösung von 0.34 g (0.88 mmol) Chlorbis(ethylen)rhodium(I), dimer in 15 mL THF hinzu. Eine sofortige Farbänderung von orange nach grün tritt ein. Das Reaktionsgemisch wird weitere zwei Stunden bei -30 °C gerührt und über Nacht im Kühlschrank bei -30 °C aufbewahrt. Anschließend wird bei -30 °C das Lösungsmittel abgezogen und das Rohprodukt aus Toluol umkristallisiert. Eine Lösung in THF/Methylenchlorid ergibt bei -30 °C nach 2 Tagen rote Kristalle. C₄₄H₅₈ClLi₂N₈O₂Rh * 2 CH₂Cl₂ (1053.08); Kristallographische Daten: Kristallfarbe/-habitus: orange Prismen; Kristallgröße: 0.31x0.09x0.07 mm³; Kristallsystem: monoklin; Raumgruppe: P2(1); Gitterkonstanten: a = 11.757(8), b = 20.046(19), c = 11.939(8) Å; β = 114.098(5)°, V = 2568.6(3) Å³; Formeleinheit: Z = 2; ρ_{ber} = 1.362 Mg/cm³; 9711 Reflexe gemessen, 5546 symmetrieunabhängige Reflexe, [I>2sigma(I)], R=0.0779, wR²(alle Daten) = 0.1976, 589 Parameter.

Die durch die NN(R)-Liganden (beispielhaft **1a** bis **1i)** stabilisierten Metallkomplexe (beispielhaft **2a-d, 3a-b, 4a, 5a-b** und **6a**) sind hochaktive und - selektive Katalysatoren. Als Beispiel für katalytische Reaktionen wird die Hydrierung von Ketonen untersucht.

### Hydrierungsbeispiele:

### Allgemeine Angaben:

Die Ansatzgröße betrug in der Regel 1 mmol. Um ungenaue Einwaagen kleinster Substanzmengen zu vermeiden wurden Stammlösungen unter inerten Bedingungen (Handschuhbox / N₂) angefertigt. Zum Abmessen der benötigten Volumina dieser Stammlösungen wurden abgedichtete Fortuna-Pipetten mit dem kleinstmöglichen Volumen verwendet. Alle Operationen wurden unter striktem Ausschluss von Sauerstoff und Feuchtigkeit in einer Handschuhbox durchgeführt. Beim Ansetzten der Katalysatorreaktion wurde eine bestimmte Zugabereihenfolge eingehalten. Die Katalysen wurden bis auf einige Voroptimierungen meist mehrfach (2-4-mal) durchgeführt.

Die jeweiligen Ansätze werden in der Handschuhbox in die Probenröhrchen pipettiert und in den Autoklaven gefüllt. Die Zugabe der Additive erfolgt hierbei im Überschuss. Der Autoklav wird an die Hydrierapparatur angeschlossen, 10-15-mal mit 5-10 bar H₂ gespült und dann wird der jeweilige Wasserstoffdruck aufgepresst. Alle Hydrierexperimente erfolgten bei konstantem Wasserstoffpartialdruck, konstanter Temperatur und unter Rühren. Durch Ablassen des Wasserstoffs wird die Hydrierung nach den entsprechenden Reaktionszeiten abgebrochen und die Proben mit der jeweiligen Menge an Dodecan als interner Standard versetzt. Zur Aufarbeitung werden die Reaktionsansätze zunächst mit H₂O versetzt und anschließend mit Ether ausgeschüttelt/extrahiert. Die Proben werden mittels GC analysiert.

Nach dieser Vorschrift erfolgte die Umsetzung von Propiophenon, 4'-Methylpropiophenon, 4'-Fluorpropiophenon, α-Methylpropiophenon, 2,2-Dimethylpropiophenon, 4'-Methoxypropiophenon und Butyrophenon.

Angaben zur katalytischen Aktivität und Selektivität:

Die Katalysatorsysteme zeigen sehr gute Enantioselektivitäten in asymmetrischen Katalysen wie beispielsweise der Hydrierung von Ketonen.

Die Katalysatorsysteme erreichen ihre Effizienz bevorzugt unter Zugabe von Metallalkoxiden, wie beispielsweise KO*^{t}*Bu (Bu = C₄H₉). Neben dem angeführten Beispiel KO*^{t}*Bu wirken auch andere Metallsalze Effizienz steigernd.

### Beispiel 7a: Hydrierung von Propiophenon

**Tabelle 1. Hydrierung von Propiophenon mit 2a**

| Bsp. 7a | Kat | LM | Additiv | T | t | Umsatz | ee |
|---|---|---|---|---|---|---|---|
| Nr. | [mol %] | | | [°C] | [h] | [%] | [%] |
| 01* | 0.0312 | THF | KO*^{t}*Bu | 25 | 24 | >99 | 73 |
| 02* | 0.01 | THF | KO*^{t}*Bu | 25 | 48 | 97 | 71 |
| 03* | 0.0625 | THF | NEt₃ | 25 | 24 | 40 | 10 |
| 04* | 0.0625 | THF | KF | 25 | 24 | 33 | 22 |
| 05* | 0.01 | THF | NaO*^{t}*Bu | 25 | 72 | 69 | 65 |
| 06* | 0.01 | THF/Aceton | KN(SiMe₃)₂ | RT | 24 | 90 | >99 |
| 07** | 0.05 | THF/Aceton | KN(SiMe₃)₂ | 25 | 24 | 76 | 92 |
| 08** | 0.05 | THF/Aceton | KO*^{t}*Bu | 15 | 24 | 53 | >99 |
| 09** | 0.05 | THF/Aceton | KN(SiMe₃)₂ | 15 | 24 | 24 | >99 |
| 10** | 0.02 | Aceton | KO*^{t}*Bu | 25 | 24 | 35 | >99 |
| 11** | 0.02 | THF | KO*^{t}*Bu | RT | 48 | 44 | 99 |
| 12** | 0.05 | THF/Aceton | KO*^{t}*Bu | RT | 24 | >99 | >99 |
| 13^{a}** | 0.07 | THF/Aceton | KO*^{t}*Bu | RT | 48 | 92 | 95 |
| 14^{a}** | 0.05 | THF/Aceton | KO*^{t}*Bu | RT | 48 | 85 | 97 |

| | | | | | | | |
|---|---|---|---|---|---|---|---|
| *70 bar H₂-Druck; **20 bar H₂-Druck; RT=15-20 °C; LM=Lösungsmittel/-gemisch; ^{a} Katalysator wurde nach Syntheseweg **B** hergestellt | | | | | | | |

**Tabelle 2. Hydrierung von Propiophenon mit 2b***

| Bsp. 7a Nr. | Kat [ mol %] | LM | Additiv | T [°C] | t [h] | Umsatz [%] | ee [%] |
|---|---|---|---|---|---|---|---|
| 01 | 0.03 | THF/Aceton | KO*^{t}*Bu | 20 | 48 | 92 | >99 |
| 02 | 0.05 | THF/Aceton | KO*^{t}*Bu | 20 | 48 | >99 | 97 |

| | | | | | | | |
|---|---|---|---|---|---|---|---|
| *20 bar H₂-Druck; LM=Lösungsmittel/-gemisch; | | | | | | | |

**Tabelle 3. Hydrierung von Propiophenon mit 2c*^{,a}**

| Bsp. 7a Nr. | Kat [mol %] | LM | Additiv | T [°C] | t [h] | Umsatz [%] | ee [%] |
|---|---|---|---|---|---|---|---|
| 01 | 0.05 | THF/Aceton | KO*^{t}*Bu | 20 | 48 | 78 | 89 |
| 02 | 0.01 | THF/Aceton | KO*^{t}*Bu | 20 | 48 | 53 | >99 |

| | | | | | | | |
|---|---|---|---|---|---|---|---|
| *20 bar H₂-Druck; LM=Lösungsmittel/-gemisch; ^{a} Katalysator wurde nach Syntheseweg **B** hergestellt | | | | | | | |

**Tabelle 4. Hydrierung von Propiophenon mit 2d***

| Bsp. 7a Nr. | Kat [mol %] | LM | Additiv | T [°C] | t [h] | Umsatz [%] | ee [%] |
|---|---|---|---|---|---|---|---|
| 01 | 0.05 | THF/Aceton | KO*^{t}*Bu | 20 | 72 | 87 | 87 |
| 02 | 0.06 | THF/Aceton | KO*^{t}*Bu | 20 | 48 | 99 | 96 |

| | | | | | | | |
|---|---|---|---|---|---|---|---|
| *20 bar H₂-Druck; LM=Lösungsmittel/-gemisch; Tabelle 5. Hydrierung von Propiophenon mit **3b*** | | | | | | | |

| Bsp.7a Nr. | Kat [mol %] | LM | Additiv | T [°C] | t [h] | Umsatz [%] | ee [%] |
|---|---|---|---|---|---|---|---|
| 01 | 0.05 | THF | KO*^{t}*Bu | 20 | 24 | 98 | 59 |
| 02 | 0.05 | THF/Aceton | KO*^{t}*Bu | 20 | 24 | 98 | 95 |

| | | | | | | | |
|---|---|---|---|---|---|---|---|
| *20 bar H₂-Druck; LM=Lösungsmittel/-gemisch; | | | | | | | |

**Tabelle 6. Hydrierung von Propiophenon mit 5b***

| Bsp.7a Nr. | Kat [mol %] | LM | Additiv | T [°C] | t [h] | Umsatz [%] | ee [%] |
|---|---|---|---|---|---|---|---|
| 01 | 0.05 | THF | - | 20 | 48 | 83 | 7 |
| 02 | 0.05 | THF | KO*^{t}*Bu | 20 | 24 | 98 | 37 |
| 03 | 0.05 | THF/Aceton | KO*^{t}*Bu | 20 | 24 | 99 | 75 |

| | | | | | | | |
|---|---|---|---|---|---|---|---|
| *20 bar H₂-Druck; LM=Lösungsmittel/-gemisch; | | | | | | | |

### Beispiel 7b: Hydrierung von 4'-Methylpropiophenon

**Tabelle 7. Hydrierung von 4'-Methylpropiophenon mit 2a***

| Bsp. 7b Nr. | Kat [mol %] | LM | Additiv | T [°C] | t [h] | Umsatz [%] | ee [%] |
|---|---|---|---|---|---|---|---|
| 01 | 0.1 | THF/Aceton | KO*^{t}*Bu | RT | 48 | 98 | 98 |
| 02 | 0.07 | THF/Aceton | KO*^{t}*Bu | RT | 48 | 71 | >99 |
| 03 | 0.05 | THF/Aceton | KO*^{t}*Bu | RT | 48 | 58 | >99 |

| | | | | | | | |
|---|---|---|---|---|---|---|---|
| *20 bar H₂-Druck; RT=15-20 °C; LM=Lösungsmittel/-gemisch; | | | | | | | |

### Beispiel 7c: Hydrierung von 4'-Fluorpropiophenon

**Tabelle 8. Hydrierung von 4'-Fluorpropiophenon mit 2a***

| Bsp.7c Nr. | Kat [mol %] | LM | Additiv | T [°C] | t [h] | Umsatz [%] | ee [%] |
|---|---|---|---|---|---|---|---|
| 01 | 0.1 | THF/Aceton | KO*^{t}*Bu | RT | 24 | 82 | >99 |
| 02 | 0.05 | THF/Aceton | KO*^{t}*Bu | RT | 24 | 73 | >99 |

| | | | | | | | |
|---|---|---|---|---|---|---|---|
| *20 bar H₂-Druck; RT=15-20 °C; LM=Lösungsmittel/-gemisch; | | | | | | | |

### Beispiel 7d: Hydrierung von α-Methylpropiophenon

**Tabelle 9. Hydrierung von α-Methylpropiophenon mit 2a**

| Bsp. 7d Nr. | Kat [mol %] | LM | Additiv | T [°C] | t [h] | Umsatz [%] | ee [%] |
|---|---|---|---|---|---|---|---|
| 01* | 0.0312 | THF | KO*^{t}*Bu | 50 | 24 | >99 | 40 |
| 02* | 0.0625 | THF | KO*^{t}*Bu | 25 | 24 | >99 | 45 |
| 03* | 0.01 | THF | KO*^{t}*Bu | 25 | 48 | 98 | 17 |
| 04** | 0.05 | THF/Aceton | KN(SiMe₃)₂ | 25 | 48 | 92 | 99 |
| 05** | 0.07 | THF/Aceton | KO*^{t}*Bu | 25 | 48 | >99 | >99 |
| 06** | 0.05 | THF/Aceton | KO*^{t}*Bu | 25 | 48 | 98 | >99 |
| 07^{a}** | 0.05 | THF/Aceton | KO*^{t}*Bu | RT | 48 | 99 | 82 |

| | | | | | | | |
|---|---|---|---|---|---|---|---|
| *70 bar H₂-Druck; **20 bar H₂-Druck; RT=15-20 °C; LM=Lösungsmittel/-gemisch; ^{a} Katalysator wurde nach Syntheseweg **B** hergestellt | | | | | | | |

**Tabelle 10. Hydrierung von α-Methylpropiophenon mit 2c*^{,a}**

| Bsp.7d Nr. | Kat [mol %] | LM | Additiv | T [°C] | t [h] | Umsatz [%] | ee [%] |
|---|---|---|---|---|---|---|---|
| 01 | 0.03 | THF/Aceton | KO*^{t}*Bu | 20 | 48 | 64 | 98 |
| 02 | 0.05 | THF/Aceton | KO*^{t}*Bu | 20 | 48 | 97 | 99 |

| | | | | | | | |
|---|---|---|---|---|---|---|---|
| *20 bar H₂-Druck; LM=Lösungsmittel/-gemisch; ^{a} Katalysator wurde nach Syntheseweg **B** hergestellt | | | | | | | |

### Beispiel 7e: Hydrierung von 2,2-Dimethylpropiophenon

**Tabelle 11. Hydrierung von 2,2-Dimethylpropiophenon mit 2a**

| Bsp.7e Nr. | Kat [mol %] | LM | Additiv | T [°C] | t [h] | Umsatz [%] | ee [%] |
|---|---|---|---|---|---|---|---|
| 01* | 0.05 | THF/Aceton | KO*^{t}*Bu | RT | 48 | >99 | 89 |
| 02** | 0.05 | THF/Aceton | KO*^{t}*Bu | RT | 48 | 82 | 95 |
| 03** | 0.05 | THF/Aceton | KO*^{t}*Bu | RT | 72 | >99 | 91 |

| | | | | | | | |
|---|---|---|---|---|---|---|---|
| *20 bar H₂-Druck; **40 bar H₂-Druck; RT=15-20 °C; LM=Lösungsmittel/-gemisch; | | | | | | | |

### Beispiel 7f: Hydrierung von 4'-Methoxypropiophenon

**Tabelle 12. Hydrierung von 4'-Methoxypropiophenon mit 2a***

| Bsp.7f Nr. | Kat [mol %] | LM | Additiv | T [°C] | t [h] | Umsatz [%] | ee [%] |
|---|---|---|---|---|---|---|---|
| 01 | 0.05 | THF/Aceton | KO*^{t}*Bu | RT | 48 | 99 | >99 |

| | | | | | | | |
|---|---|---|---|---|---|---|---|
| *20 bar H₂-Druck; RT=15-20 °C; LM=Lösungsmittel/-gemisch; | | | | | | | |

### Beispiel 7g: Hydrierung von Butyrophenon

**Tabelle 13. Hydrierung von Butyrophenon mit 2a***

| Bsp.7g Nr. | Kat [mol %] | LM | Additiv | T [°C] | t [h] | Umsatz [%] | ee [%] |
|---|---|---|---|---|---|---|---|
| 01 | 0.01 | THF/Aceton | KO*^{t}*Bu | RT | 48 | 64 | >99 |
| 02 | 0.05 | THF/Aceton | KO*^{t}*Bu | RT | 24 | 99 | >99 |

| | | | | | | | |
|---|---|---|---|---|---|---|---|
| *20 bar H₂-Druck; RT=15-20 °C; LM=Lösungsmittel/-gemisch; | | | | | | | |

Die Untersuchung zur Hydrierung von α-Methyl- und 2,2-Dimethylpropiophenon zeigen, dass auch sterisch anspruchsvolle und damit schwer zu hydrierende Substrate (T. Ohkuma, et al., J. Am. Chem. Soc. 2005, 127, 8288-8289) effizient, mit sehr guten Enantioselektivitäten mit den erfindungsgemäßen Katalysatorsystemen hydriert werden können.

## Patentansprüche

1. Imidazo[1,5-*b*]pyridazin-substituierte Amino-Liganden der Formel **1**, 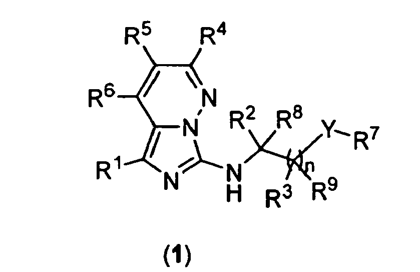 worin
n für eine ganze Zahl von 1 bis 30 steht,
Y für ein Sauerstoff- oder Schwefelatom oder eine NH-Gruppe steht und
R¹-R⁹ unabhängig voneinander einen Rest ausgewählt aus der Gruppe C₁- C₂₄ Alkyl, C₂-C₂₄ Alkenyl, C₃-C₈ Cycloalkyl, C₃-C₈ Cycloalkenyl, C₆- C₁₄ Aryl, Phenyl, Naphthyl, Fluorenyl, C₃-C₈ Heteroalkyl, C₃-C₈ Heterocycloalkyl, C₂-C₁₃ Heteroaryl, Hydroxy, Acyl, Silyl, Boryl, darstellen können und wobei die Reste R¹, R⁴, R⁵, R⁶ und R⁷ unabhängig voneinander auch einen Wasserstoffrest darstellen können und wobei R² und R⁸ Wasserstoffreste sein können, wenn mindestens einer der Reste R³ und R⁹ kein Wasserstoffsubstituent ist oder R³ und R⁹ verschiedene Reste darstellen und wobei R³ und R⁹ Wasserstoffreste sein können, wenn mindestens einer der Reste R² und R⁸ kein Wasserstoffsubstituent ist oder R² und R⁸ verschiedene Reste darstellen und bei denen die genannten Substituenten R¹ bis R⁹ jeweils ein oder mehrere weitere Substituenten besitzen können, und wobei zwei benachbarte Substituenten R¹, R⁴, R⁵ oder R⁶ oder die Substituenten R², R³, R⁷, R⁸ und R⁹ auch verbrückt sein können.

2. Verbindungen nach Anspruch 1, **dadurch gekennzeichnet, dass**
R¹ einen Rest ausgewählt aus der Gruppe bestehend aus Phenyl, Alkyl, *tert*.-Butyl oder Heteroaryl, die auch weitere Substituenten tragen können, darstellt, und/oder
R⁴ und R⁶ unabhängig voneinander Reste ausgewählt aus der Gruppe bestehend aus Phenyl, Alkyl und Trifluormethyl, darstellen, und/oder
R⁵ ein Wasserstoffsubstituent ist, und/oder
R² und R⁸ Reste darstellen, die ausgewählt sind aus der Gruppe der aromatischen Reste, Phenyl, der aliphatisch verzweigten oder unverzweigten Alkylreste, Alkylphenylreste oder Wasserstoffsubstituenten und/oder
R³ und R⁹ Wasserstoffsubstituenten oder aliphatisch verzweigte oder unverzweigte Alkyl- oder Hydroxyalkylreste, Hydroxyreste, Ketoreste und/oder
R⁷ ein Wasserstoffsubstituent oder Acetylrest ist.

3. Verbindungen nach Anspruch 1, **dadurch gekennzeichnet, dass** die Gruppe -(CR³R⁹)ₙ- eine Methylen-, Ethylen-, Propylen- oder Butylengruppe darstellt und
R¹, R², R⁴, R⁵ R⁶ unabhängig voneinander einen Rest ausgewählt aus der Gruppe C₁-C₂₄ Alkyl, C₂-C₂₄ Alkenyl, C₃-C₈ Cycloalkyl, C₃-C₈ Cycloalkenyl, C₆-C₁₄ Aryl, Phenyl, Naphthyl, Fluorenyl, C₃-C₈ Heterocycloalkyl, C₂-C₁₃ Heteroaryl, darstellen können und wobei die Reste R¹, R⁴, R⁵ und R⁶ unabhängig voneinander auch einen Wasserstoffrest darstellen können und
R⁷ und R⁸ einen Wasserstoffrest darstellen.

4. Verbindungen nach einem der Ansprüche 1 bis 3, **dadurch gekennzeichnet, dass** die Verbindungen der Formel 1 optisch aktiv sind.

5. Verbindungen nach Anspruch 4, **dadurch gekennzeichnet, dass** die Verbindungen der Formel 1 enantiomerenangereichert sind.

6. Verbindungen nach einem der Ansprüche 1 bis 5, **dadurch gekennzeichnet, dass** sie in neutraler, ionischer oder ein- oder mehrfach deprotonierter Form vorliegen.

7. Komplexverbindung enthalten mindestens einen Liganden nach einem der Ansprüche 1 bis 6 und mindestens ein Metallatom oder -ion.

8. Komplexverbindungen gemäß Anspruch 7, **dadurch gekennzeichnet, dass** die Komplexverbindungen mindestens ein Übergangsmetallatom oder -ion ausgewählt aus der Gruppe Palladium, Platin, Rhodium, Ruthenium, Osmium, Iridium, Kobalt, Eisen, Nickel, Kupfer, Silber und/oder Gold enthalten.

9. Komplexverbindungen gemäß Ansprüchen 7 und 8, **dadurch gekennzeichnet, dass** die Komplexverbindungen mindestens ein Alkali- bzw. Erdalkalimetallatom oder -ion ausgewählt aus der Gruppe Lithium, Natrium, Kalium, Magnesium, Calcium, und/oder mindestens ein Übergangsmetallatom oder -ion ausgewählt aus der Gruppe Scandium, Yttrium, Lanthan, Titan, Zirkonium, Hafnium, oder mindestens ein Lanthanoidatom oder -ion enthalten.

10. Komplexverbindungen gemäß einem der Ansprüche 7 bis 9, **dadurch gekennzeichnet, dass** die Komplexverbindung eine Verbindung der Formeln **2-6** ist, 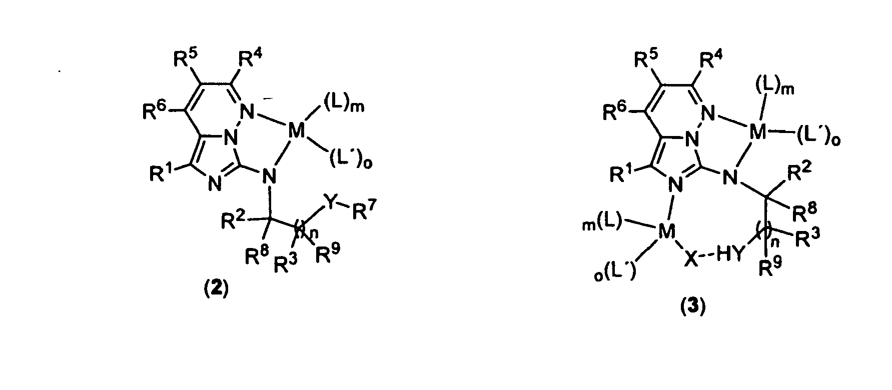 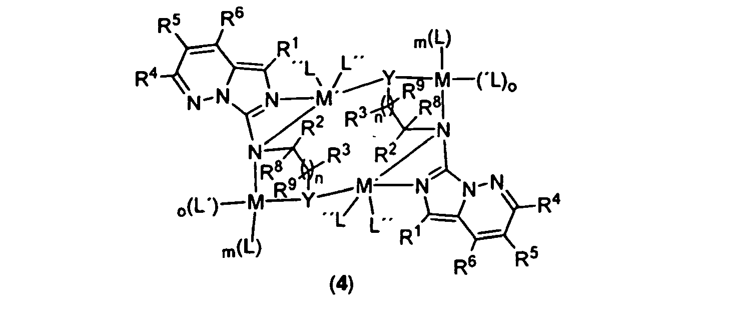 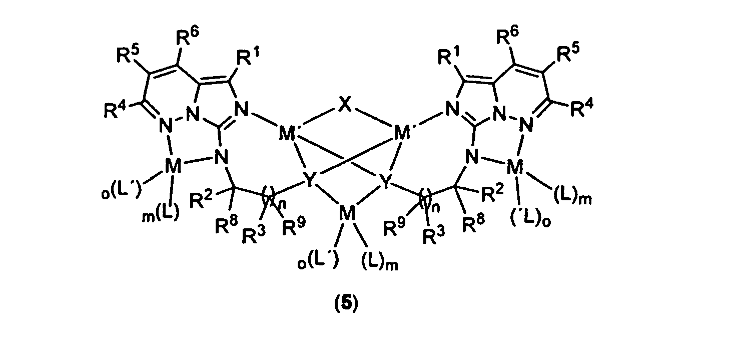 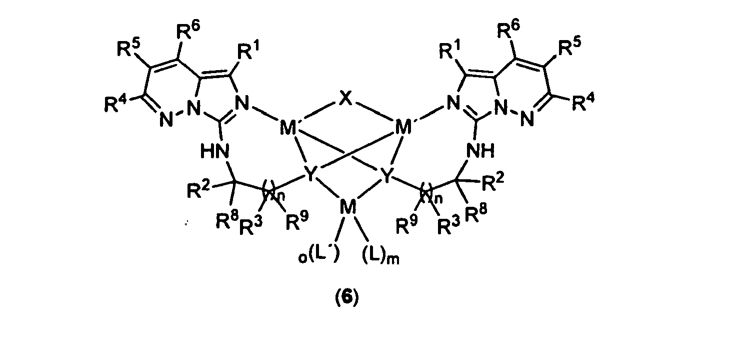 wobei
n, Y und die Substituenten R¹ bis R⁹ die in Anspruch 1 genannte Bedeutung haben und
m und o unabhängig voneinander 0 oder 1 sein können und
X für ein Halogenid-Liganden steht und
M und M' jeweils ein Metall darstellt und
L, L' und L" unabhängig voneinander für einzähnige Liganden oder L und L' zusammengenommen für einen zweizähnigen Liganden stehen.

11. Komplexverbindung nach einem der Ansprüche 7-10, **dadurch gekennzeichnet, dass** der Katalysator in Verbindung mit einem Additiv, ausgewählt aus der Gruppe der Metalloxidverbindungen, Alkali- oder Erdalkalimetall- alkylate, allylate, -arylate, -phosphate, -hydride, -halogenide, der Silyloxyverbindungen, Aminverbindungen oder der Alkalimetalle vorliegt.

12. Verwendung einer Komplexverbindung nach den Ansprüchen 7 bis 11 als Katalysator zur asymmetrischen Hydrierung.

13. Verfahren zur asymmetrischen Synthese von Alkoholen, umfassend die enantioselektive Hydrierung von Ketonen mit einer Komplexverbindung als Katalysator entsprechend den Ansprüchen 7-12.

## Claims

1. Imidazo[1,5-*b*]pyridazine-substituted amino ligands
of the formula **1**, 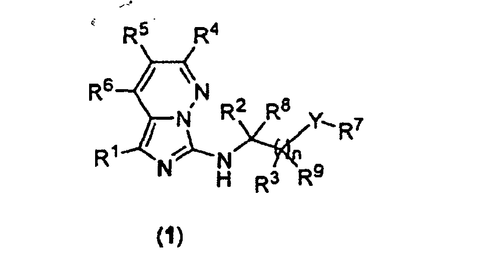 where
n is an integer from 1 to 30,
Y is an oxygen or sulphur atom or an NH group and
R¹-R⁹ can each be, independently of one another, a radical selected from the group consisting of C₁-C₂₄-alkyl, C₂-C₂₄-alkenyl, C₃-C₈-cycloakyl, C₃-C₈-cycloalkenyl, C₆-C₁₄-aryl, phenyl, naphthyl, fluorenyl, C₃-C₈-heteroalkyl, C₃-C₈- heterocycloalkyl, C₂-C₁₃-heteroaryl, hydroxy, acyl, silyl, boryl and the radicals R¹, R⁴, R⁵, R⁶ and R⁷ can also be, independently of one another, a hydrogen radical and R² and R⁸ can be hydrogen radicals when at least one of the radicals R³ and R⁹ is not a hydrogen substituent or R³ and R⁹ are different radicals and R³ and R⁹ can be hydrogen radicals when at least one of the radicals R² and R⁸ is not a hydrogen substituent or R² and R⁸ are different radicals and where said substituents R¹ to R⁹ can each have one or more further substituents and two adjacent substituents R¹, R⁴, R⁵ or R⁶ or the substituents R², R³, R⁷, R⁸ and R⁹ can also be bridged.

2. Compounds according to Claim 1, **characterized in**
**that**
R¹ is a radical selected from the group consisting of phenyl, alkyl, *tert*-butyl or heteroaryl which can each also bear further substituents and/or
R⁴ and R⁶ are each, independently of one another, a radical selected from the group consisting of phenyl, alkyl and trifluoromethyl and/or
R⁵ is a hydrogen substituent and/or
R² and R⁸ are radicals selected from the group consisting of aromatic radicals, phenyl, aliphatically branched or unbranched alkyl radicals, alkylphenyl radicals and hydrogen substituents and/or
R³ and R⁹ are hydrogen substituents or aliphatic- ally branched or unbranched alkyl or hydroxyalkyl radicals, hydroxy radicals, keto radicals and/or
R⁷ is a hydrogen substituent or acetyl radical.

3. Compounds according to Claim 1, **characterized in that** the group -(CR³R⁹)ₙ- is a methylene, ethylene, propylene or butylene group and
R¹, R², R⁴, R⁵ , R⁶ can each be, independently of one another, a radical selected from the group consisting of C₁-C₂₄-alkyl, C₂-C₂₄-alkenyl, C₃-C₈-cycloalkyl, C₃-C₈-cycloalkenyl, C₆-C₁₄- aryl, phenyl, naphthyl, fluorenyl, C₃-C₈- heterocycloalkyl, C₂-C₁₃-heteroaryl, and the radicals R¹, R⁴, R⁵ and R⁶ can also be, independently of one another, a hydrogen radical and
R⁷ and R⁸ are each a hydrogen radical.

4. Compounds according to any of Claims 1 to 3, **characterized in that** the compounds of the formula 1 are optically active.

5. Compounds according to Claim 4, **characterized in that** the compounds of the formula 1 are enantiomerically enriched.

6. Compounds according to any of Claims 1 to 5, **characterized in that** they are present in uncharged, ionic or monodeprotonated or multiply deprotonated form.

7. Complex containing at least one ligand according to any of Claims 1 to 6 and at least one metal atom or ion.

8. Complexes according to Claim 7, **characterized in that** the complexes contain at least one transition metal atom or ion selected from the group consisting of palladium, platinum, rhodium, ruthenium, osmium, iridium, cobalt, iron, nickel, copper, silver and gold.

9. Complexes according to Claims 7 and 8, **characterized in that** the complexes contain at least one alkali metal or alkaline earth metal atom or ion selected from the group consisting of lithium, sodium, potassium, magnesium, calcium, and/or at least one transition metal atom or ion selected from the group consisting of scandium, yttrium, lanthanum, titanium, zirconium, hafnium,
or at least one lanthanide atom or ion.

10. Complexes according to any of Claims 7 to 9, **characterized in that** the complex is a compound of the formulae **2-6,** 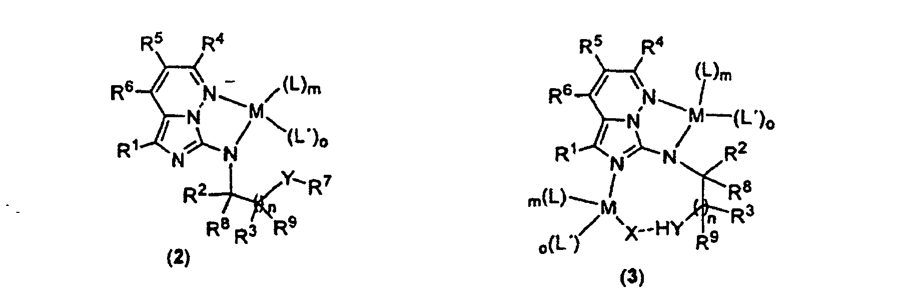 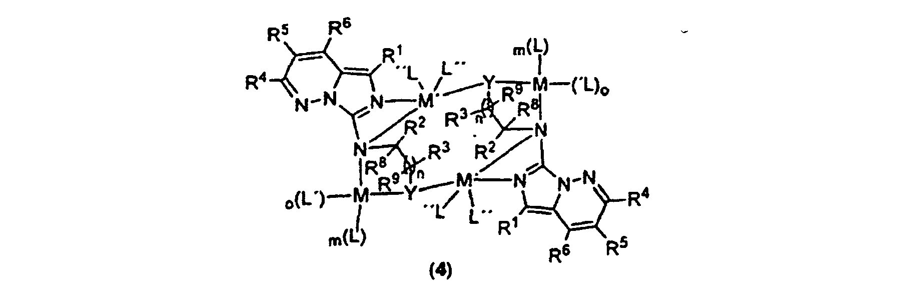 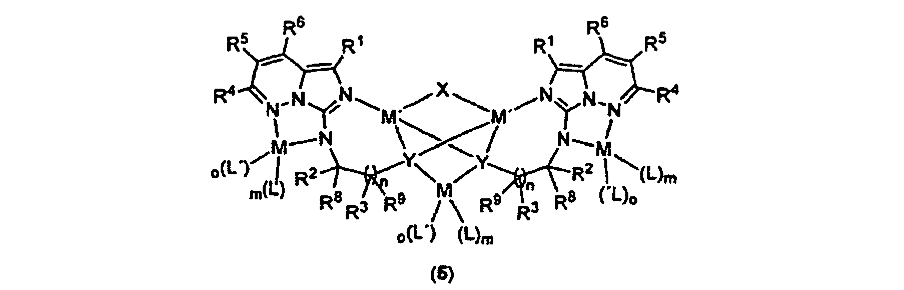 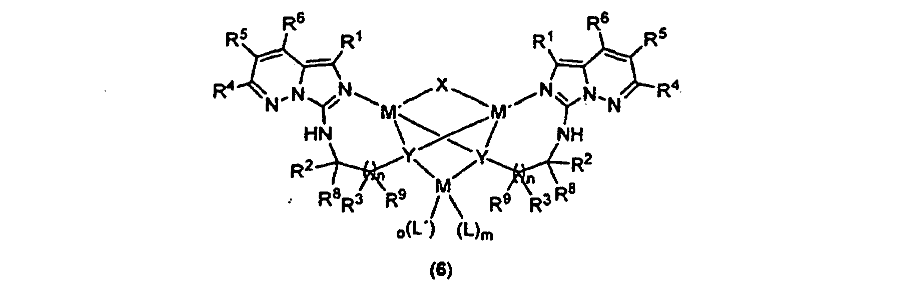 where
n, Y and the substituents R¹ to R⁹ are as defined in Claim 1 and
m and o can each be, independently of one another, 0 or 1 and
X is a halide ligand and
M and M' are each a metal and
L, L' and L" are each, independently of one another, a monodentate ligand or L and L' together form a bidentate ligand.

11. Complex according to any of Claims 7-10, **characterized in that** the catalyst is present in combination with an additive selected from the group consisting of metal oxide compounds, alkali metal or alkaline earth metal alkylates, allylates, arylates, phosphates, hydrides, halides, silyloxy compounds, amine compounds and alkali metals.

12. Use of a complex according to any of Claims 7 to 11 as catalyst for asymmetric hydrogenation.

13. Process for the asymmetric synthesis of alcohols, which comprises the enantioselective hydrogenation of ketones using a complex according to any of Claims 7-12 as catalyst.

## Revendications

1. Aminoligands substitués par imidazo[1,5-b]pyridazin de formule 1, 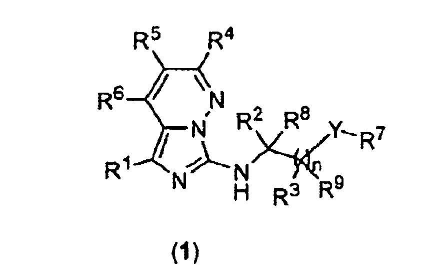 où
n représente un nombre entier de 1 à 30,
Y représente un atome d'oxygène ou de soufre ou un groupe NH et
R¹ à R⁹ peuvent représenter indépendamment l'un de l'autre un radical choisi parmi le groupe alkyle en C₁ à C₂₄, alcényle en C₂ à C₂₄, cycloalkyle en C₃ à C₈, cycloalcényle en C₃ à C₈, aryle en C₆ à C₁₄, phényle, naphthyle, fluorényle, hétéroalkyle en C₃ à C₈, hétérocycloalkyle en C₃ à C₈, hétéroaryle en C₂ à C₁₃, hydroxyle, acyle, silyle, boryle, et
sachant que les radicaux R¹, R⁴, R⁵, R⁶ et R⁷ peuvent aussi représenter indépendamment l'un de l'autre un radical hydrogène et
sachant que R² et R⁸ peuvent être des radicaux hydrogène quand au moins un des radicaux R³ et R⁹ n'est pas un substituant hydrogène ou que R³ et R⁹ représentent des radicaux différents et
sachant que R³ et R⁹ peuvent être des radicaux hydrogène, quand au moins un des radicaux R² et R⁸ n'est pas un substituant hydrogène ou que R² et R⁸ représentent des radicaux différents et
chez lesquels les substituants cités R¹ à R⁹ peuvent posséder respectivement un ou plusieurs autres substituants, et
sachant que deux substituants voisins R¹, R⁴, R⁵ ou R⁶ ou les substituants R², R³, R⁷, R⁸ et R⁹ peuvent aussi être pontés.

2. Composés selon la revendication 1, **caractérisés en ce que**
R¹ représente un radical choisi parmi le groupe consistant en un phényle, un alkyle, un tert.-butyle ou un hétéroaryle, qui peuvent aussi porter d'autres substituants, et/ou
R⁴ et R⁶ représentent indépendamment l'un de l'autre des radicaux choisis parmi le groupe consistant en un phényle, un alkyle et un trifluorométhyle, et/ou
R⁵ est un substituant hydrogène, et/ou
R² et R⁸ représentent des radicaux qui sont choisis parmi le groupe des radicaux aromatiques, du phényle, des radicaux alkyles aliphatiques ramifiés ou non ramifiés, des radicaux alkylphényle ou des substituants hydrogène et/ou
R³ et R⁹ sont des substituants hydrogène ou des radicaux alkyles ou hydroxyalkyles aliphatiques ramifiés ou non ramifiés, des radicaux hydroxyles, des radicaux cétone et/ou
R⁷ est un substituant hydrogène ou un radical acétyle.

3. Composés selon la revendication 1, **caractérisés en ce que** le groupe -(CR³R⁹)ₙ- représente un groupe méthylène, éthylène, propylène ou butylène et
R¹, R², R⁴, R⁵, R⁶ peuvent représenter indépendamment l'un de l'autre un radical choisi parmi le groupe alkyle en C₁ à C₂₄, alcényle en C₂ à C₂₄, cycloalkyle en C₃ à C₈, cycloalcényle en C₃ à C₈, aryle en C₆ à C₁₄, phényle, naphthyle, fluorényle, hétérocycloalkyle en C₃ à C₈, hétéroaryle en C₂ à C₁₃, et sachant que les radicaux R¹, R⁴, R⁵ et R⁶ peuvent aussi représenter indépendamment l'un de l'autre un radical hydrogène et
R⁷ et R⁸ représentent un radical hydrogène.

4. Composés selon l'une quelconque des revendications 1 à 3, **caractérisés en ce que** les composés de la formule 1 sont optiquement actifs.

5. Composés selon la revendication 4, **caractérisés en ce que** les composés de la formule 1 sont enrichis en énantiomères.

6. Composés selon l'une quelconque des revendications 1 à 5, **caractérisés en ce qu'**ils se présentent sous la forme neutre, ionique ou une ou plusieurs fois déprotonée.

7. Composés complexes contenant au moins un ligand selon une des revendications 1 à 6 et au moins un atome ou un ion métallique.

8. Composés complexes selon la revendication 7, **caractérisés en ce que** les composés complexes contiennent au moins un atome ou un ion d'un métal de transition choisi parmi le groupe palladium, platine, rhodium, ruthénium, osmium, iridium, cobalt, fer, nickel, cuivre, argent et/ou or.

9. Composés complexes selon les revendications 7 et 8, **caractérisés en ce que** les composés complexes contiennent au moins un atome d'un métal ou d'un ion alcalin, respectivement alcalinoterreux, choisi parmi le groupe lithium, sodium, potassium, magnésium, calcium, et/ou au moins un atome ou un ion d'un métal de transition choisi parmi le groupe scandium, yttrium, lanthane, titane, zirconium, hafnium, ou au moins un atome ou un ion lanthanide.

10. Composés complexes selon l'une quelconque des revendications 7 à 9, **caractérisés en ce que** le composé complexe est un composé des formules 2 à 6, 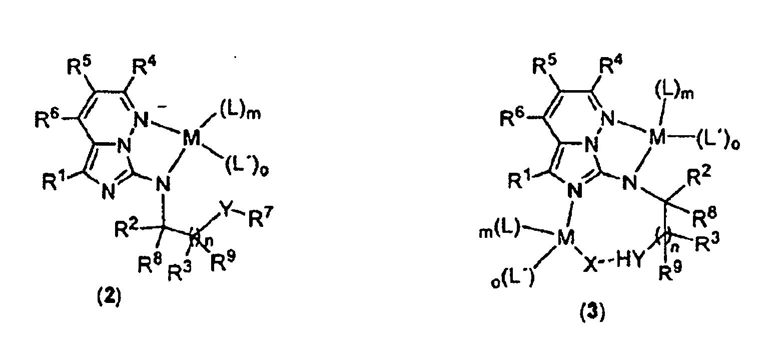 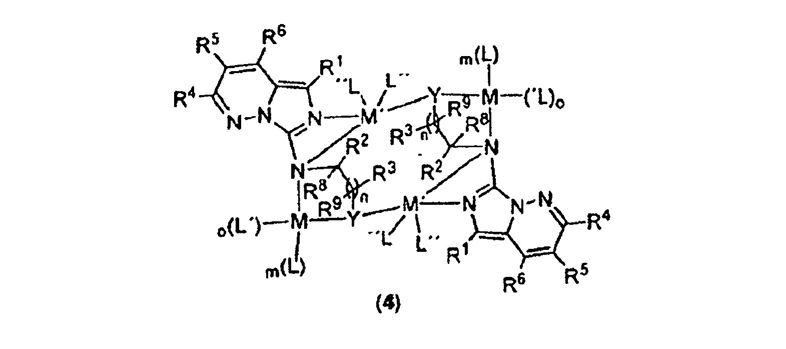 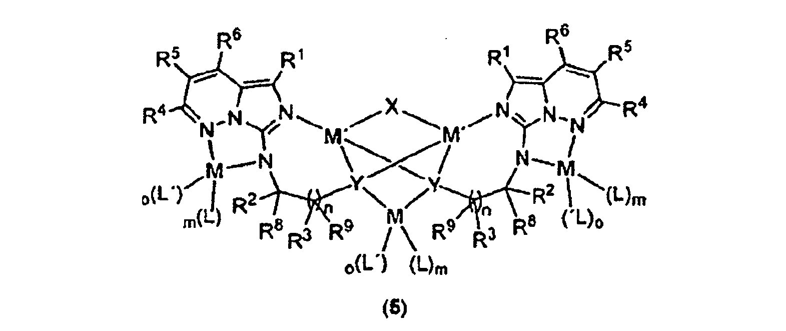 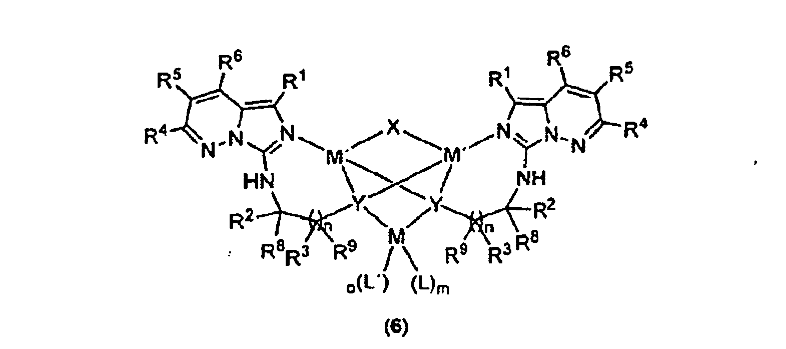 dans lesquelles n, Y et les substituants R¹ à R⁹ ont la signification spécifiée dans la revendication 1 et
m et o peuvent être indépendamment l'un de l'autre 0 ou 1 et
X représente un ligand halogénure et
M et M' représentent chacun un métal et
L, L' et L" représentent indépendamment l'un de l'autre des ligands monodenté ou L et L' représentent conjointement un ligand bidenté.

11. Composé complexe selon l'une quelconque des revendications 7 à 10, **caractérisé en ce que** le catalyseur est présent en liaison avec un additif, choisi parmi le groupe des composés d'oxydes métallique, des alkylates, des allylates, des arylates, des phosphates, des hydrures, des halogénures de métaux alcalins ou alcalinoterreux, des composés silyloxyles, des composés amines ou des métaux alcalins.

12. Utilisation d'un composé complexe selon l'une quelconque des revendications 7 à 11 comme catalyseur pour l'hydrogénation asymétrique.

13. Procédé pour la synthèse asymétrique d'alcools, comprenant l'hydrogénation énantiosélective de cétones avec comme catalyseur un composé complexe conformément aux revendications 7 à 12.
